# EUROPEAN PATENT SPECIFICATION

(11) **EP 4 121 028 B1**
(45) Date of publication and mention of the grant of the patent: **04.11.2026**
(21) Application number: 21770886.6
(22) Date of filing: 16.03.2021
(51) Int. Cl.: A61K 9/16, A61K 31/216, A61K 31/192, A61K 31/55

(54) **CONTINUOUS MELT-COATING OF ACTIVE PHARMACEUTICAL INGREDIENTS USING SURFACTANTS FOR DISSOLUTION ENHANCEMENT**
KONTINUIERLICHE SCHMELZBESCHICHTUNG VON PHARMAZEUTISCHEN WIRKSTOFFEN MIT TENSIDEN ZUR AUFLÖSUNGSVERBESSERUNG
ENROBAGE PAR FUSION CONTINU DE PRINCIPES ACTIFS PHARMACEUTIQUES À L'AIDE DE TENSIOACTIFS POUR L'AMÉLIORATION DE LA DISSOLUTION

(30) Priority: 17.03.2020 US 202062990578 P
(43) Date of publication of application: 25.01.2023
(73) Proprietor: Rutgers, the State University of New Jersey, New Brunswick, NJ 08901 (US)
(72) Inventor: MUZZIO, Fernando J., New Brunswick, NJ 08901 (US); LIU, Zhanjie, New Brunswick, NJ 08901 (US); CALLEGARI, Gerardo, New Brunswick, NJ 08901 (US); HAUSNER, Douglas, New Brunswick, NJ 08901 (US)
(74) Representative: Hindles Limited
(86) International application number: PCT/US2021/022533
(87) International publication number: WO 2021/188517

(56) References cited:
- WO-A1-2007/086846
- WO-A1-2010/094471
- WO-A1-2015/193485
- JP-A- 2018 507 180
- US-A1- 2009 202 649
- US-A1- 2010 055 133
- US-A1- 2017 157 049
- US-A1- 2020 024 736
- BECKER KARIN ET AL: "Solvent-Free Melting Techniques for the Preparation of Lipid-Based Solid Oral Formulations", ENVIRONMENTAL SCIENCE AND POLLUTION RESEARCH, SPRINGER BERLIN HEIDELBERG, BERLIN/HEIDELBERG, vol. 32, no. 5, 19 March 2015 (2015-03-19), pages 1519 - 1545, XP035444577, ISSN: 0724-8741, [retrieved on 20150319], DOI: 10.1007/S11095-015-1661-Y

## Description

### CROSS-REFERENCE TO RELATED APPLICATION

This application claims priority to U.S. Provisional Patent Application Serial No. 62/990,578, filed March 17, 2020.

### FIELD

The disclosed technology generally relates to the field of pharmaceutical manufacturing, particularly compositions and manufacturing methods for continuous melt-coating of active pharmaceutical ingredients (APIs) with surfactants to produce drug products having enhanced dissolution properties. Also more generally, the disclosed technology provides a convenient means for modifying the surface of granular matter, which is useful in many industries, including food, construction, agriculture, catalyst manufacture, metallurgy, ceramics, etc.

### BACKGROUND

A large percentage of APIs and new chemical entities have been reported as being poorly water-soluble. In general, poorly water-soluble APIs present challenges such as poor absorption and low bioavailability, which makes it is difficult to deliver the API into a subject's blood stream. The US Pharmacopeia describes the solubility of drugs in terms of the number of milliliters of solvent in which one gram of solute will dissolve. Typically, drugs defined as "poorly soluble" are those that require more than 1 ml of solvent per 10 mg of solute. The Biopharmaceutics Classification System (BCS) divides drugs into the following four groups with respect to solubility and permeability: Class I (high solubility, high permeability), Class II (low solubility, high permeability), Class III (high solubility, low permeability), and Class IV (low solubility, low permeability). According to the BCS, a drug substance is considered "poorly soluble" or of low solubility when more than 250 mL of an aqueous solution in a pH range of 1.2 to 6.8 at 37±1°C is required to solubilize the highest single therapeutic dose. Permeability is evaluated with respect to the extent of absorption of a drug from human pharmacokinetic studies. A drug is considered "highly permeable" when its absolute bioavailability is greater than or equal to 85%. Among the four BCS groups, drugs in Classes II and IV exhibit poor aqueous solubility, resulting in poor bioavailability. Such poorly soluble drugs also often exhibit uneven absorption, with the degree of unevenness being influenced by factors such as dose level, patient satiety, and drug form, as well as a number of other parameters along the manufacturing process affecting the final product properties.

US 2009/0202649A1 describes pharmaceutical formulations comprising fenofibrate. It also describes stable and bioavailable pharmaceutical formulations comprising fenofibrates. Further, this document describes processes for preparing the compositions and/or formulations of fenofibrate and their methods of use, treatment and administration.

JP 2018507180A explains that the dissolution of Active Pharmaceutical Ingredients in polymeric melts plays an important role in the manufacturing of drugs that use polymers as excipients. The dissolution kinetics is essential for designing the processing equipment, describing the operating conditions, and defining material properties, for example, the appropriate API-polymer(s) pair. In one embodiment, the solubility of ketoprofen (KTO) in Soluplus^{®}, Kollidon^{®} VA64, Kollidon^{®} SR and a combination of three; was analyzed under Hot Melt Extrusion (HME) processing conditions. Thermal characterization techniques show that a single phase amorphous solid solution (only one Tg) was achieved by HME at 120° C and 70 rpm. The sample's stability was analyzed for 4 weeks and the single phase amorphous solid solution was maintained during that time. An extended release profile of KTO was achieved, releasing 100% of KTO in 12 h.

Karin Becker et al, 'Solvent-Free Melting Techniques for the Preparation of Lipid-Based Solid Oral Formulations', Pharm Res (2015) 32: 1519-1545 describes lipid excipients applied for numerous purposes such as taste masking, controlled release, improvement of swallowability and moisture protection. The document intends to give a review of the published literature on experiences, formulations and challenges and to show possibilities for future developments in this field. It also serves as a guide for selecting the best excipients and manufacturing techniques for the development of a product with specific properties using solvent free melt processing.

In the field of pharmaceutical drug development, oral administration is a preferred route for drug delivery due to several advantages such as low cost, pain avoidance, patient convenience, and safety. Thus, there is often a need to improve solubility and bioavailability, including oral bioavailability, of pharmaceutical drug products containing poorly soluble APIs. Prior methods of addressing challenges associated with poorly soluble APIs include micronization, amorphization, spray drying and hot melt extrusion, but such methods tend to have significant disadvantages, such as high cost, difficulty of implementation, instability, and dosage limitations. For example, micronization can cause a drug substance to be poorly compressible and poorly flowable, and reduction in particle size can increase the tendency of the drug to undesirably agglomerate. Spray drying and hot melt extrusion are complex, expensive to implement and operate, and can result in dosage forms that contain large amounts of excipients, thus limiting the amount of drug that can be loaded into the product units.

Additionally, solid pharmaceutical products, e.g., tablets and capsules, are often manufactured in a batch processing manner, whereby the drug products are made according to a manufacturing sequence that includes a series of unit operations, such as blending, granulating, milling, tableting, etc. In general, batch manufacturing is a step-by-step process requiring multiple pieces of equipment to implement all the unit operations. Each unit operation on any given batch typically must finish before the next unit operation can be applied. In contrast, in continuous manufacturing, only a small portion of the production lot is being processed at any given time, the material flows continuously from each unit operation to the next, and multiple unit operations proceed simultaneously. Because continuous processes are operated at or near a steady state, and because only a small amount of material is being processed at any given time, continuous processes typically require smaller space, provide higher productivity, better quality, better product uniformity, lower labor costs, and more stable and reliable processes than their batch counterparts. Thus, there is a need to further develop continuous processes for manufacturing pharmaceutical drug products, including solid pharmaceutical dosage forms.

The disclosed technology addresses one or more of the foregoing needs by providing a process for continuously melt-coating APIs with surfactants to produce melt-coated API particles that provide for enhanced dissolution of drug products formulated therefrom.

### SUMMARY

The invention is set out in the appended set of claims.

The present disclosure relates to a surprisingly effective and efficient method of enhancing dissolution of poorly soluble drugs by employing a combination of simultaneously applied shear and heat in a continuous process. In general, the poorly soluble drug is combined, optionally pre-blended, with a small amount of a low melting point surfactant, and the combination is then continuously processed in a processor at temperatures close to the melting point of the surfactant. It has been surprisingly discovered that this process causes the surfactant to soften, melt, or partially melt so as to coat the outer surface of at least a portion of the drug particles. The presence of the surfactant on the surface of the drug promotes wetting, and greatly enhances dissolution of the poorly soluble drug. In some embodiments of the disclosed technology, APIs can also be melt coated with low-melting-point substances to control their release rate, mask unpleasant taste or smell, improve chemical and/or physical stability of the product, reduce moisture sensitivity or light sensitivity, maintain a desired pH, and/or a myriad other applications.

The disclosed method provides a substantial improvement over prior methods; it is much easier to implement and much less expensive. Further, since the disclosed method does not rely on dispersing the drug in a matrix of another ingredient (as is the case, for example, in spray drying, hot melt extrusion, or other co-processing methods) it is possible to make tablets, capsules and other dosage forms that are almost entirely comprised of the drug substance and that contain only a minimal amount of excipient(s). Consequently, the disclosed method in its various embodiments is suitable for achieving high drug loading of a unit dose. Moreover, the process is performed continuously, and thus provides significant advantages over batch processing techniques.

In one aspect, the disclosed technology relates to a continuous process for melt-coating an active pharmaceutical ingredient, including: (a) introducing an active pharmaceutical ingredient (API) and a surfactant into a twin-screw processor; and (b) continuously heating and shearing the API and surfactant in the twin-screw processor at a processing temperature that is below the melting point of the surfactant and no lower than 10°C below the melting point of the surfactant so as to form melt-coated API particles including API particles with at least a partial coating of surfactant, wherein melt-coated API particles are formed, in which the surfactant coats 30% or more of the outer surface of the API particles, as determined by SEM images, wherein the surfactant has a melting point of at least 10°C lower than the API melting point, and wherein the melt-coated API particles comprise at least 90wt% API based on the total weight of the melt-coated API particles. In one embodiment, the melt-coated API particles have faster dissolution as compared to a physical mix of the API and the surfactant, as determined by the time required to dissolve 80% of the drug substance in the melt-coated particles, when tested using any one of the following: (1) a USP II apparatus, 900 ml vessel, 75 RPM, using deionized water; (2) a USP II apparatus, 900 ml vessel, 75 RPM, using pH 2 simulated gastric fluid; or (3) a USP II apparatus, 900 ml vessel, 75 RPM, using pH 6.8 buffer. In one embodiment, the continuous process is operated under closed loop control using a combination of sensors, controllers, and actuators to maintain the process within a desired range of operating parameters. In another embodiment, the continuous processor is an extruder, blender, mixer, or kneader. In another embodiment, the API has an equilibrium solubility of less than 50 mg/ml in de-ionized water at 25 degrees centigrade.

In another embodiment, the API includes one or more of ibuprofen, carbamazepine, fenofibrate, acetaminophen, indomethacin, flufenamic acid, imatinib, erlotinib hydrochloride, vitamin D, a steroid, estrodial, and/or a non-steroidal anti-inflammatory drug. The surfactant has a melting point of at least 10 degrees centigrade lower than the API melting point. In another embodiment, the surfactant includes one or more of poloxamer, polyoxyethylene stearate, cetylpyridinium chloride, polysorbate, and glyceryl monostearate. In another embodiment, the process further includes: (c) continuously formulating the melt-coated API particles into a finished pharmaceutical drug product. In another embodiment, the finished pharmaceutical drug product is a solid oral dosage form. In another embodiment, the solid oral dosage form is selected from a tablet, a capsule, a powder, and a granulate. In another embodiment, step (c) further includes combining the melt-coated API particles with one or more pharmaceutically acceptable excipients selected from carriers, modified release agents, fillers, extenders, binders, humectants, disintegrating agents, absorption accelerators, wetting agents, pH modifiers, absorbents, lubricants, coloring agents, and diluents.

In another aspect, the disclosed technology relates to a pharmaceutical drug product including melt-coated API particles prepared by the continuous process disclosed herein. In one embodiment, the pharmaceutical drug product includes melt-coated API particles prepared by the continuous process disclosed herein, and at least one pharmaceutically acceptable excipient. In one embodiment, individual product units have faster dissolution as compared to an individual product unit that differs only by having been made from a physical mix of the API and the surfactant, as determined by the time required to release 80% of the API in the product, when tested using any one of the following: (1) a USP II apparatus, 900 ml vessel, 75 RPM, using deionized water; (2) a USP II apparatus, 900 ml vessel, 75 RPM, using pH 2 simulated gastric fluid; or (3) a USP II apparatus, 900 ml vessel, 75 RPM, using pH 6.8 buffer.

In another aspect, the disclosed technology relates to melt-coated active pharmaceutical ingredient (API) particles prepared by continuously feeding, heating and shearing API and surfactant in a twin-screw processor at a temperature that is below the melting point of the surfactant and no lower than 10°C below the melting point of the surfactant so as to form melt-coated API particles, wherein: the melt-coated API particles include at least a partial coating of the API by the surfactant; the melt-coated API particles are formed, in which the surfactant coats 30% or more of the outer surface of the API particles as determined by SEM images; the surfactant has a melting point of at least 10°C lower than the API melting point; the melt-coated API particles include surfactant in an amount of at least 1 wt % based on the total weight of the melt-coated API particles; and the melt-coated API particles have enhanced dissolution as compared to a physical mix of the API and the same proportional amount of surfactant. The melt-coated API particles include API in an amount of at least 90 wt%, at least 95 wt%, or at least 99 wt% based on the total weight of the melt-coated API particles.

In another aspect, the disclosed technology relates to a solid oral dosage form including the melt-coated API particles and at least a pharmaceutically acceptable excipient, wherein: the solid oral dosage form includes API in an amount of at least 1 wt% (e.g., at least 5 wt%, at least 10 wt%, at least 20 wt%, at least 30 wt%, at least 40 wt% or more) based on the total weight of the solid oral dosage form; and upon dissolution, the solid oral dosage form releases the API at least 20% faster than a solid oral dosage form that differs only by having been made from a physical mix of the API and the surfactant, when tested using any one of the following: (1) a USP II apparatus, 900 ml vessel, 75 RPM, using deionized water; (2) a USP II apparatus, 900 ml vessel, 75 RPM, using pH 2 simulated gastric fluid; or (3) a USP II apparatus, 900 ml vessel, 75 RPM, using pH 6.8 buffer. In one embodiment, the solid oral dosage form is selected from a tablet, a capsule, a powder, a granulate, and an implant.

In yet another embodiment, the API particles are continuously melt-coated by simultaneous application of heat and shear to mixtures of API with low-melting-point substances with desirable properties, so as to accomplish one or more of the following: decrease the moisture sensitivity of the API, decrease the light sensitivity of the API, decrease the cohesion of the API particles, improve the flow properties of the API particles, mask unpleasant taste or odor of the API particles, provide and maintain a desired pH in the environment surrounding the API particle, and other desirable attributes that can be achieved by choice of the low melting point substance. In one embodiment, a low melting point wax or fatty acid can be used to make the API surfaces less likely to react when exposed to moisture. Low melting point waxes, alcohols, and fragrances can be used to mask taste or smell. Low melting point radical killer substances can be used to prevent oxidative degradation. Many other such applications of melt coating are contemplated as well.

A variety of additional aspects will be set forth in the description that follows. The aspects can relate to individual features and to combinations of features. It is to be understood that both the foregoing general description and the following detailed description are exemplary and explanatory only and are not restrictive of the broad inventive concepts upon which the embodiments disclosed herein are based. The person of ordinary skill in the art, with a practical understanding of pharmaceutical formulation, pharmaceutical manufacturing, and continuous processing would know how to use the invention disclosed here, in combination with routine experiments, to achieve additional variations in composition of matter, manufacturing process, and product formulation methods.

### BRIEF DESCRIPTION OF THE DRAWINGS

The accompanying drawings, which are incorporated herein and constitute part of this specification, are illustrative of particular embodiments of the present disclosure and do not limit the scope of the present disclosure. The drawings are not to scale and are intended for use in conjunction with the explanations in the following detailed description.

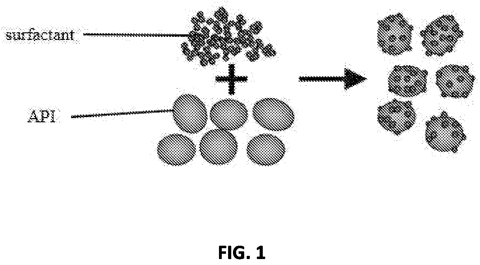

**FIG. 1** shows an example illustration of the disclosed concept of producing melt-coated API from a pre-blend of API and surfactant.
**FIG. 2** is a graph showing dissolution profiles related to Ibuprofen and poloxamer as described in Example 2.
**FIG. 3** is a graph showing dissolution profiles related to Ibuprofen and poloxamer as described in Example 2.
**FIG. 4** is a graph showing dissolution profiles related to Ibuprofen and poloxamer as described in Example 2.
**FIG. 5** is a graph showing tablet dissolution profiles related to Ibuprofen and Poloxamer 407 as described in Example 3.
**FIG. 6** is a graph showing dissolution profiles related to Ibuprofen and polyoxyethylene stearate as described in Example 4.
**FIG. 7** is a graph showing dissolution profiles related to carbamazepine and cetylpyridinium chloride as described in Example 5.
**FIG. 8** is a graph showing capsule dissolution profiles related to carbamazepine and cetylpyridinium chloride as described in Example 5.
**FIGS. 9A-D** are SEM images of tablets prepared and analyzed as described in Example 6. Fig. 9A shows an SEM image of Fenofibrate : Poloxamer407=10:1 physical mixture (200X magnification). Fig. 9B shows an SEM image of Fenofibrate : Poloxamer407=10:1 physical mixture (500X magnification). Fig. 9C shows an SEM image of Fenofibrate : Poloxamer407=10:1 untreated (25°C). Fig. 9D shows an SEM image of Fenofibrate: Poloxamer407=10:1 thermo-treated. Fig. 9E shows an SEM image of Fenofibrate : Poloxamer407=10:0.5 thermo-treated.
**FIG. 10** is a graph showing dissolution of fenofibrate (FNF) powder, as described in Example 6.
**FIG. 11** is a graph showing the effect of screw-filling level on melt-coating, as described in Example 6.
**FIG. 12** is a graph showing the effect of melt coating at different barrel temperatures (API : surfactant = 10:0.5), as described in Example 6.
**FIGS. 13A-B** are bar graphs showing the porosity and disintegration time of tablets, as described in Example 6. Fig. 13A is a bar graph showing tablet porosity (%). Fig. 13B is a bar graph showing disintegration time (s).
**FIG. 14** is a graph showing the dissolution of fenofibrate (FNF) tablets, as described in Example 6.

### DETAILED DESCRIPTION

The following discussion omits or only briefly describes conventional features of the disclosed technology that are apparent to those skilled in the art. Reference to various embodiments does not limit the scope of the claims attached hereto. Additionally, any examples set forth in this specification are intended to be non-limiting and merely set forth some of the many possible embodiments for the appended claims. Further, particular features described herein can be used in combination with other described features in each of the various possible combinations and permutations. A person of ordinary skill in the art would know how to use the instant invention, in combination with routine experiments, to achieve other outcomes not specifically disclosed in the examples or the embodiments.

Unless otherwise specifically defined herein, all terms are to be given their broadest possible interpretation including meanings implied from the specification as well as meanings understood by those skilled in the art and/or as defined in dictionaries, treatises, etc. Unless defined otherwise, all technical and scientific terms used herein have the same meaning as commonly understood by one of ordinary skill in the art in the field of the disclosed technology. It must also be noted that, as used in the specification and the appended claims, the singular forms "a," "an" and "the" include plural referents unless otherwise specified, and that the terms "comprises" and/or "comprising," when used in this specification, specify the presence of stated features, elements, and/or components, but do not preclude the presence or addition of one or more other features, steps, operations, elements, components, and/or groups thereof. Additionally, methods, equipment, and materials similar or equivalent to those described herein can also be used in the practice or testing of the disclosed technology.

### Melt-Coated API Particles

As used herein, the term "melt-coated API particles" refers to particles of an active pharmaceutical ingredient (API) having at least a partial coating of surfactant that was melted onto the outer surfaces of the API particles according to the continuous melt-coating process disclosed herein. Any suitable, pharmaceutically acceptable drug or pro-drug substance may be used in connection with the disclosed technology. Further, one or more different APIs (e.g., 1, 2, 3 or more APIs) may be melt-coated with one or more different substances having similar functions (e.g., 1, 2, 3 or more surfactants) or having different functions (e.g., a surfactant, a chemical stabilizer, and a taste masking ingredient). Moreover, it is also understood and know in the field that a given substance can have multiple functions, and those functions might be expressed to different extents under various situations. Examples of substances with multiple functions include magnesium stearate (MgSt) (a lubricant, a glidant, and a hydrophobic material), hydroxypropyl methylcellulose (HPMC) (a binder, a wetting agent, and a controlled release agent), many forms of starch (wet binders compression binders, fillers, and disintegrants), etc.

Specific APIs disclosed herein are provided for illustrative purposes only and do not limit the scope of the disclosed technology. In general, the API used in the continuous melt-coating process should be chemically and physically stable under relevant experimental conditions, and soluble to a significant extent in different types of solvents. In some embodiments, the API is suitably soluble in a volatile organic solvent. In some embodiments, the API is suitably soluble in water, while in other embodiments, the API is poorly soluble in water, e.g., the API solubility is less than 10 mg./ml. Non-limiting examples of APIs that may be used in connection with the disclosed technology include ibuprofen, carbamazepine, fenofibrate, acetaminophen, indomethacin, flufenamic acid, imatinib, flufenamic acid, erlotinib hydrochloride, vitamin D, steroids, estrodial, other non-steroidal anti-inflammatory drugs (NSAIDs), and combinations thereof.

In a broader context, substances other than APIs can also be melt coated to modify their surface properties and their bulk attributes. The need to modify powder properties is ubiquitous in many industries, and the convenient continuous melt coating methods disclosed here can be conveniently adapted by the skilled artisan.

Specific surfactants disclosed herein are provided for illustrative purposes only and do not limit the scope of the disclosed technology. Any suitable, pharmaceutically acceptable surfactant(s) may be used in connection with the disclosed technology so long as the melting point of the surfactant is at least 10°C lower than the melting point of the API with which it is to be combined, and neither the surfactant nor the API experience substantial degradation when exposed to the temperatures and shear rates required to achieve melt coating. In some embodiments, the melting point of the surfactant is at least 15°C lower, at least 20°C lower, or at least 25°C lower than the melting point of the API. In some embodiments, the difference between the melting point of the surfactant and the higher melting point of the API is 15°C to 150°C, 20°C to 150°C, 15°C to 100°C, or 20°C to 125°C.

The surfactant may be amphoteric, non-ionic, cationic or anionic. Non-limiting examples of suitable surfactants include: cetylpyridinium chloride, sodium lauryl sulfate, monooleate, sorbitan monooleate, monolaurate, monopalmitate, monostearate or another ester of polyoxyethylene sorbitane or polyethylene glycol, diethylene glycol monostearate, glyceryl monostearate, sodium dioctylsulfosuccinate, lecithin, stearylic alcohol, cetostearylic alcohol, cholesterol, polyoxyethylene ricin oil, macrogolglycerol ricinoleate, macrogolglycerol hydroxystearate, polyoxyl 35 castor oil, polyoxyl castor oil such as polyoxyl 40 hydrogenated castor oil, hydrogenated polyoxyethylene fatty acid glycerides, pluronic surfactants such as poloxamers of different molecular weights (e.g., poloxamer 124, poloxamer 188, poloxamer 237, poloxamer 338, poloxamer 407), block copolymers of poly(ethylene oxide) and poly(propylene oxide), inulin, inutec, benzethanium chloride, docusate sodium, polyoxyethylene sorbitan fatty acid esters, polysorbate (e.g., polysorbate 80, polysorbate 60), vitamin E derivatives, polyoxyethylene alkyl ethers, polyoxyethylene stearates, saturated polyglycolyzed glycerides, fatty amine oxides, fatty acid alkanolamides, poly(oxyethylene)-block-poly(oxypropylene) copolymers, and combinations thereof.

Melt-coated API particles disclosed herein include at least 90 wt% API, such as at least 92 wt%, at least 95 wt%, at least 96 wt%, at least 97 wt%, at least 98 wt%, or at least 99 wt% API, based on the total weight of the melt-coated API particle.

### Pharmaceutical Dosage Forms

One or more surfactants can advantageously increase the rate of dissolution of the melt-coated API particles by facilitating wetting, and can also increase the maximum drug concentration of a finished pharmaceutical drug product produced therefrom by eliminating the need to transform the API into an amorphous solid dispersion, i.e., by spray drying, hot melt extrusion, or other techniques that disperse API molecules within a matrix of another substance. In some embodiments, pharmaceutical dosage forms formulated from melt-coated API particles disclosed herein include at least 10 wt% API, such as at least 15 wt%, at least 20 wt%, at least 25 wt%, at least 30 wt%, at least 35 wt%, at least 40 wt%, at least 45 wt%, at least 50 wt%, at least 55 wt%, at least 60 wt%, at least 65 wt%, at least 70 wt%, at least 75 wt%, at least 80 wt%, at least 85 wt%, or at least 90 wt% API, based on the total weight of the pharmaceutical dosage form. In general, pharmaceutical dosage forms formulated from melt-coated API particles disclosed herein are more highly soluble than their counterparts where ingredients are blended without simultaneous application of shear and heat.

In some embodiments, pharmaceutical dosage forms formulated from melt-coated API particles disclosed herein include less than or equal to 10 wt % surfactant, such as less than or equal to 9 wt%, less than or equal to 8 wt%, less than or equal to 7 wt%, less than or equal to 6 wt%, less than or equal to 5 wt%, less than or equal to 4 wt%, less than or equal to 3 wt%, less than or equal to 2 wt%, less than or equal to 1 wt% surfactant, based on the weight of the API in the pharmaceutical dosage form.

As disclosed herein, finished pharmaceutical drug products, such as solid oral dosage form pharmaceutical compositions, are suitable for administration to a subject, such as a human or other mammal. Non-limiting examples of solid oral dosage forms include tablets, capsules containing melt-coated API as described herein, optionally with other ingredients, capsules comprising a plurality of mini-tablets, powders, and granulations, and other dosage forms that are manufactured from powders and granules. Non-limiting examples of tablets include sublingual molded tablets, buccal molded tablets, sintered tablets, compressed tablets, chewable tablets, freeze-dried tablets, soluble effervescent tablets, and pellets. Non-limiting examples of capsules, in which a solid dosage form of the drug is enclosed within a hard or soft soluble container or shell, include hard gelatin capsules, soft gelatin capsules, and non-gelatin capsules. In some embodiments, the finished solid oral dosage form may be modified to achieve a desired timing of API release - e.g., a dosage form that provides immediate release, sustained release, controlled release, extended release, partial immediate and partial delayed release, and combinations thereof.

The disclosed methods can also be used in the manufacture of non-oral products where a mixture of APIs and other solid ingredients is useful, including but not limited to the manufacture of inhalants, implantable and injectable solid compositions, vascular stents, ocular implants, and the like.

In preparing a pharmaceutical drug product, including a finished solid oral dosage form, the melt-coated API particles may be blended with one or more pharmaceutically acceptable excipients. Non-limiting examples of such excipients include: carriers, such as cellulose or substituted cellulose materials, sodium citrate or dicalcium phosphate; fillers or extenders, such as starch-based materials, microcrystalline cellulose, lactose, sucrose, glucose, mannitol, and/or silicic acid; binders, such as hydroxypropyl cellulose, hydroxypropyl methylcellulose, carboxymethyl-cellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; absorption accelerators, such as quaternary ammonium compounds and additional surfactants, such as poloxamer and sodium lauryl sulfate; wetting agents, such as cetyl alcohol, glycerol monostearate, and non-ionic surfactants; absorbents, such as kaolin and bentonite clay; lubricants, such as talc, calcium stearate, magnesium stearate, solid polyethylene glycols, sodium lauryl sulfate, zinc stearate, sodium stearate, stearic acid, and mixtures thereof; coloring agents; controlled release agents, such as crospovidone, ethyl cellulose, poly(ethylene oxide), alkyl-substituted celluloses, crosslinked polyacrylic acids, xanthan gum, guar gum, carrageenan gum, locust bean gum, gellan gum, methyl cellulose, hydroxypropyl methyl cellulose, hydroxyethyl cellulose, ethyl cellulose, sodium carboxymethyl cellulose, sodium alginate, gelatin, modified starches, co-polymers of carboxyvinyl polymers, co-polymer of acrylates, co-polymers of oxyethylene and oxypropylene and mixtures thereof; diluents; and other additives, such as paraffin and high molecular weight polyethylene glycols. In mentioning these materials, the use of the term "such us" means that the mentioned materials are simply examples belonging to a more extensive class. Also, as it is known in the art, most pharmaceutical ingredients have more than one useful function, and the mention of any one ingredient in any one of the above examples is not meant to exclude the use of that material for a different purpose. For example, as mentioned, starch can be a filler, a binder, and a disintegrant, and many other materials can also be used in more than one way.

In some embodiments, the disclosed method eliminates several expensive excipients from the formulation of the finished dosage form, thus advantageously lowering cost and eliminating sources of variability that can cause quality problems in finished products.

### Continuous Melt-Coating Process

In general, the continuous melt-coating process disclosed herein includes the steps of: combining particles of at least one API with at least one surfactant to form a blend, and subjecting the blend to simultaneous shear and heat in order to form melt-coated API particles. Not wishing to be bound by theory, it is nonetheless believed that the simultaneous application of heat and shear causes localized melting of the particles of the low melting point substance at points where these particles are in direct contact with API particles. This promotes the adhesion of the particles of the low melting point substance to the surface of the API particles. Presence of surfactant particles on the surface of the API particles makes the combined particles easier to wet. The melt-coated API particles thus produced ("treated") exhibit substantially enhanced dissolution as compared to uncoated API particles (i.e., API alone), and also as compared to a mixture of the same API and the same proportion of the same surfactant. As used herein, the term "physical mix" refers to a combination of API(s) and surfactant(s) that has not been subjected to shear and heat near the melting point of the surfactant according to the continuous melt-coating process disclosed herein.

In some embodiments, the majority of each particle is coated with surfactant. In some embodiments, a finished dosage form prepared from melt-coated API contains surfactant in a total amount of about 1-10 wt%, such as about 1-8 wt%, about 1-6 wt%, about 1-5 wt%, about 1-4 wt%, about 1-3 wt%, about 2-10 wt%, about 2-8 wt%, about 2-6 wt%, about 2-5 wt%, about 2-4 wt%, about 3-10 wt%, about 3-8 wt%, about 3-6 wt%, about 3-5 wt%, about 4-10 wt%, about 4-8 wt%, or about 4-6 wt%, based on the total weight of the API in the finished dosage form.

Continuous manufacturing, in contrast to traditional batch processing, allows for the manufacturing of drug products from raw materials in a single continuous fashion such that the output is maintained at a consistent rate with no need to stop production. As a result, the disclosed technology is capable of efficiently providing homogenous pharmaceutical drug products containing large amounts of API in a robust, readily controlled, and commercially valuable manufacturing process.

In the disclosed process, continuous melt-coating can be either a stand-alone process for manufacturing melt-coated API, or can be part of a larger integrated continuous manufacturing line, either for producing API, or for manufacturing pharmaceutical products containing melt-coated API. Continuous manufacturing methods can provide significant technical and business advantages relative to batch methods. In general, continuous manufacturing methods are more robust and controllable. They achieve the same production rates as batch processes in much smaller and thus less capital-intensive equipment, which also requires less space to operate. They also facilitate automation that can be used to achieve significant improvements in product quality and process reliability. By combining melt-coating and continuous manufacturing, the disclosed methods achieve benefits afforded by both technologies and may be used as a rapid development platform to prepare clinical supplies and to introduce new drugs to market, or to manufacture those products at higher qualities and lower cost.

Further, the disclosed method allows for an integrated technology for continuous melt-coating that is designed and optimized based on a deeper understanding of the main components of the manufacturing system, helping to promote adoption of modern methodologies across an essential industry that at the present time often uses empirical methods and batch processes as its main development and manufacturing paradigm. The continuous manufacturing processes described herein may include sensing and control capabilities, such that the process is continuously monitored by various sensors, controllers, and actuators to maintain the continuous process and the resulting products within the desirable operating range of process parameters and product quality attributes. Measurements collected from sensors can be used in conjunction with controllers and actuators arranged in a closed loop system, using feedback, feed forward, and other configurations to control the performance of the process and the quality of the manufactured products.

The disclosed method also provides one or more significant advantages that make it very useful as a commercial method for drug product development and manufacturing. For example, the method is seemingly easy to perform, whereby a finished drug product may be made by melt-coating surfactant(s) onto API particles, optionally mixing the melt coated API with other ingredients, including other APIs, compacting the melt-coated API into tablets or filling it into capsules, vials, blister packs, or the like. This process can eliminate expensive processing steps, such as crystallization, drying, and milling of the drug material, batch blending with excipients, dry or wet granulation, drying or wet sizing of the granulation, sizing of the dry granulation, mixing the granulation with extragranular ingredients, and the like. By simplifying the process, the method significantly accelerates product development.

The disclosed method is also readily up- or down-scalable, facilitating manufacturing at both clinical trial and commercial scales and enabling rapid scale-up (or scale-down) and scale-out of manufacturing rates to meet changing market demands. In its simplest form, a continuous process enables the operator to make as much, or as little product as desired simply by changing the length of time the process is operated.

In some embodiments, the disclosed technology relates to processes of continuously manufacturing a finished pharmaceutical drug product using melt-coated API particles made by either a continuous or a non-continuous (e.g., batch) process. In such embodiments, the material being processed in the continuous process flows through multiple simultaneous unit operations, including feeding melt-coated API into feeder, optionally combining the melt-coated API with one or more pharmaceutically acceptable excipients in a continuous processor, and compounding the mixture into a desired solid oral dosage form. Non-limiting examples of other suitable finishing steps include filling the mixture into capsules, vials, or aerosol blisters, or compressing the mixture into tablets.

The disclosed continuous manufacturing process may include a series of unit operations and online testing equipment. In one embodiment, the process includes a first feeder for delivering API, a second feeder for delivering surfactant, an optional blender for pre-blending API and surfactant, an optional mill for milling either API alone or pre-blended API and surfactant, and a twin-screw processor capable of subjecting the API and surfactant to heat and shear simultaneously.

The first feeder continuously dispenses API particles. The second feeder continuously dispenses surfactant. In some embodiments in which more than one type of API and/or more than one type of surfactant is used, additional corresponding feeders and/or blenders may also be employed. Alternatively, the first feeder may contain more than one type of API and/or the second feeder may contain more than one type of surfactant. The API and surfactant are continuously fed into the processor. In some embodiments, the API and surfactant are continuously fed into the processor in the absence of a solvent. In some embodiments, the one or more API and one or more surfactant are continuously fed into the processor in the absence of a solvent and/or in the absence of any other materials (e.g., excipients, such as carriers, fillers, extenders, binders, humectants, disintegrating agents, absorption accelerators, wetting agents, absorbents, lubricants, coloring agents, and diluents). In some embodiments, the API feeder and/or the surfactant feeder is operated at room temperature. In some embodiments, more than one thermal processor is used as part of the integrated continuous manufacturing process.

In some embodiments, the API and surfactant may be directed into a batch vessel for pre-blending, after which the pre-blend may be directed into the processor. In some embodiments, the API or the optionally prepared pre-blend may be milled, and then directed into the processor.

In the processor, API and surfactant are subjected simultaneously to both continuous shear stress and heat. As used herein, "shear stress" refers to a stress in a material in multiple directions, both parallel and orthogonal to the tangent to the surface of the API particles. In general, the shear stresses applied in the processor are converted to additional heat through friction and compression, which helps to melt or soften the surfactant without melting or otherwise physically or chemically modifying the API. In some embodiments, the disclosed continuous process minimizes or eliminates the formation of API aggregates. In some embodiments, the disclosed continuous process minimizes or eliminates the need for granulation. The processor parameters are selected so as to efficiently provide a melt-coating of surfactant on the outer surface of the API particles. In some embodiments, the majority of the outer surface of the API particles is coated with surfactant(s).

Non-limiting examples of suitable processors include extruders, such as twin screw extruders, blenders, mixers, kneaders, and other shearing devices. A heat source, such as a heat exchanger, may be provided as an integrally formed part of the processor. Optionally, heat may be included in the continuous process as a separate device immediately prior to the application of shear. In one embodiment, the processor comprises a heated barrel or jacket. In one embodiment, the process comprises an extruder having multiple screws. When an extruder contains multiple screws, the screws may be arranged for co-rotation and/or counter-rotation. In some embodiments, the processor includes a combination of kneading and conveying elements (e.g., alternating kneading and conveying elements) wherein the kneading elements apply shear forces and the conveying elements transfer the API, surfactant, and melt-coated API particles through the processor. In some embodiments, the processor includes a rotating shearing device, such as a screw extruder, impeller, agitator, blade, or the like. The rotating shearing device may rotate at a speed of 100 rpm to 1000 rpm, such as 100 rpm to 700 rpm, 150 rpm to 500 rpm, or 150 rpm to 300 rpm.

The processor may be operated in a selected mode, such as a partially filled or starved mode, which can be adjusted by adjusting the rate(s) at which the mixture of API and surfactant are fed into the processor. In a partially filed mode, a continuous volume of API, surfactant, and melt-coated API particles is maintained inside the processor during the melt-coating process, wherein the continuous volume is 25-75% of the total volume of the processor. In a starved mode, the API and surfactant are fed into the processor at a slower rate than the rate at which the API particles are coated with the surfactant. In some embodiments, the API and surfactant are fed into the processor at a rate of 100 g/h to 50 kg/h, such as 150 g/h to 1 kg/h, or 200 g/h to 300 g/h. In one embodiment, the API and the surfactant are not premixed, but they are added at a carefully controlled relative rate, and mixing happens within the extruder.

The API and surfactant are processed in the processor at a temperature below the melting point of the surfactant and no lower than 10°C below the melting point of the surfactant, and below the melting point of the API(s). When more than one surfactant is used, the processing temperature is below the highest melting point of the surfactants. When the melting point of an API or surfactant is defined in terms of a range of temperature values, the average temperature value should be relied upon for purposes of making and using the disclosed technology described herein. The processing temperature is selected so as to melt, partially melt, or soften the surfactant, thus causing the surfactant to effectively smear or nanosmear onto the outer surfaces of the API particles. Higher temperatures are unnecessary and should be avoided.

The processing temperature is below the melting point of the surfactant and no lower than 10°C below the melting point of the surfactant. For example, the processing temperature may range from below the melting point of the surfactant to a temperature that is 10°C, 9°C, 8°C, 7°C, 6°C, 5°C, 4°C, 3°C, 2°C, or 1°C below the melting point of the surfactant.

Processing temperatures ranging between any of the foregoing temperatures are contemplated herein as well. For example, the processing temperature may range from 10°C below the melting point of the surfactant to 5°C below the melting point.

The disclosed technology also contemplates the use of a temperature profile along the process, where, for example, a lower processor temperature may be used near the entrance of the materials to enable their homogenization prior to melting and coating, a higher temperature may then be applied in a central region of the processor to achieve the desired degree of coating, and a lower temperature might be used near the exit of the processor to cool down the coated particles and prevent them from sticking to one another or to equipment surfaces.

The time spent by the API or a detectable tracer material inside the processor during the disclosed continuous melt-coating process is the mean residence time (MRT). It has been surprisingly discovered that the simultaneous application of heat and shear in a continuous processor enables substantial coating of the API particles with surfactant in very short processing times. The very short exposure to heat and shear substantially prevents thermal degradation of the API and/or the surfactant that would likely occur in batch processes requiring much longer times or higher temperatures to achieve comparable degrees of melt coating. In some embodiments, the MRT is less than or equal to 30 seconds, less than or equal to 1 minute, less than or equal to 2 minutes, less than or equal to 3 minutes, less than or equal to 4 minutes, less than or equal to 5 minutes, less than or equal to 6 minutes, less than or equal to 7 minutes, less than or equal to 8 minutes, less than or equal to 9 minutes, less than or equal to 10 minutes, or less than or equal to 15 minutes. In some embodiments, the MRT is 1 second to 30 seconds, 1 second to 1 minute, 1 second to 2 minutes, 1 second to 3 minutes, 1 second to 4 minutes, 1 second to 5 minutes, 1 second to 6 minutes, 1 second to 7 minutes, 1 second to 8 minutes, 1 second to 9 minutes, 1 second to 10 minutes, 30 seconds to 1 minute, 30 sec to 2 minutes, 30 sec to 3 minutes, 30 sec to 4 minutes, 30 seconds to 5 minutes, or 30 seconds to 10 minutes.

After melt-coating, the melt-coated API particles are collected from the processor. In some embodiments, the collected melt-coated API particles may then be formulated into a finished drug product. Non-limiting examples of finished drug products include solid oral dosage forms such as tablets, capsules, powders, and granulates. The finished drug product may be further provided in appropriate packaging, such as but not limited to a blister pack, a bottle, or vial.

Dissolution testing may be performed to determine the release drug profile of the melt-coated API particles and also of finished dosage forms formulated from the melt-coated API particles. Among other devices suitable for dissolution testing known to those of ordinary skill in the art, a 708-DS, 8-spindle, 8-vessel USP dissolution apparatus type II (paddle), with automated online UV-Vis measurement (Agilent Technologies) could be used for such measurements. In some embodiments, the melt-coated API particles and finished drug products formulated therefrom may have a dissolution drug release profile such as, but not limited to:
- An immediate release profile, where either the melt-coated API particles or the finished drug product formulated from the melt-coated API particles releases at least 80% of the API in less than 45 minutes, at least 80% of the API in less than 30 minutes, at least 80% of the API in less than 15 minutes, at least 80% of the API in less than 10 minutes, when tested in a USP II apparatus using 900 ml of simulated gastric fluid with pH<2, 50 RPM, and 37°C.
- An immediate release profile, where either the melt-coated API particles or the finished drug product formulated from the melt-coated API particles releases at least 80% of the API in less than 45 minutes, at least 80% of the API in less than 30 minutes, at least 80% of the API in less than 15 minutes, at least 80% of the API in less than 10 minutes, when tested in a USP II apparatus using 900 ml of de-ionized water, 50 RPM, and 37°C.
- A sustained release profile, where either the melt-coated API particles or the finished drug product formulated from the melt-coated API particles releases less than 80% of the API after 60 minutes, or after 120 minutes, or after 240 minutes, or after 360 minutes, or after 480 minutes, or after 1440 minutes, when tested in a USP II apparatus using 900 ml of de-ionized water, 50 RPM, and 37°C.
- A sustained release profile, where either the melt-coated API particles or the finished drug product formulated from the melt-coated API particles releases less than 80% of the API after 60 minutes, or after 120 minutes, or after 240 minutes, or after 360 minutes, or after 480 minutes, or after 1440 minutes, when tested in a USP II apparatus using 900 ml of pH=6.8 buffer, 50 RPM, and 37°C.
- A delayed release profile, where less than 5% of the API, or less than 10 % of the API, is released from either the melt-coated API particles or the finished drug product formulated from the melt-coated API particles after one hour, when tested in a USP II apparatus using 900 ml of simulated gastric fluid with pH<2, 50 RPM, and 37°C.
- The time period for 50% of the API to be released from either the melt-coated API particles or the finished drug product formulated from the melt-coated API particles is half, or less than half, of the time period required for 50% of the API to be released from a physical mix of the API and surfactant or a finished drug product formulated from a physical mix of the API and surfactant, respectively.

### EXAMPLES

The disclosed technology is next described by means of the following non-limiting examples. The use of these and other examples anywhere in the specification is illustrative only, and in no way limits the scope and meaning of the invention or of any exemplified form. Likewise, the invention is not limited to any particular preferred embodiments described herein. The invention is therefore to be limited only by the terms of the claims. Efforts have been made to ensure accuracy with respect to values presented (e.g., amounts, temperature, etc.), but some experimental error and deviation should be accounted for. Unless indicated otherwise, parts are parts by weight, molecular weight is weight average molecular weight, temperature is in degrees Centigrade, and pressure is at or near atmospheric.

### Example I

This example describes the preparation and analysis of (i) an API (ibuprofen) alone and (ii) physically mixed with surfactant (poloxamer) as compared to (iii) ibuprofen melt-coated with poloxamer in accordance with the disclosed technology. Ibuprofen has a hydrophobic surface, lower water solubility, and a melting point of 75°C to 78°C. In this example, the following three types of ibuprofen were used:
Ibuprofen 1: spheroideal shaped particles, small particle size (D50 ≈ 30 micrometers)
Ibuprofen 2: spheroideal shaped particles, larger particle size (D50 ≈ 50 micrometers)
Ibuprofen 3: needle shaped particles, larger particle size (D50 ≈ 60 micrometers)

Poloxamer generally functions as a dispersing agent, emulsifying agent, solubilizing agent, wetting agent, and lubricant. Many grades of Poloxamer have a melting point of 52°C to 57°C. The specific grade used here has a particle size distribution characterized by D10 (for example, D10 = 13.24 microns); D50 (for example, D50 = 39.68 microns); and D90 (for example, D90 = 72.48 microns), as determined by laser light scattering or some other suitable method. Poloxamer is available in various grades, as indicated in Table 1 below based on its chemical structure.

### Chemical Structure of Poloxamer:

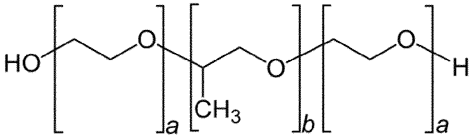

**Table 1**

| **Poloxamer** | **Physical Form** | **a** | **b** | **Average molecular weight** |
|---|---|---|---|---|
| 124 | Liquid | 12 | 20 | 2090-2360 |
| 188 | Solid | 80 | 27 | 7680-9510 |
| 237 | Solid | 64 | 37 | 6840-8830 |
| 338 | Solid | 141 | 44 | 12700-17400 |
| 407 | Solid | 101 | 56 | 9840-14600 |

In this example, each of Poloxamer 188 (P188) and Poloxamer 407 (P407) were used in an amount of either 5 wt% or 10 wt% based on the total combined weight of the API and surfactant.

Pre-blends of poloxamer and ibuprofen were prepared using a small V-blender with a 1 qt vessel size, operated at 25 rpm for 15 minutes, without an intensifier bar. Continuous melt-coating of the poloxamer onto the ibuprofen particles was carried out in an 11 mm Thermo Fisher twin screw extruder having 3 or 4 kneading zones, with 3 kneading elements in each zone, at a processing temperature of 35°C and an operational speed of 150 rpm or 300 rpm. The screw configurations are shown below, where "C" represents conveying elements and "K" represents kneading elements:

| | | | | | | | | | |
|---|---|---|---|---|---|---|---|---|---|
| Screw Configuration A | 35 °C | | | | | | | | |
| | **C** | **K** | **C** | **K** | **C** | **K** | **C** | **K** | **C** |
| | 35 °C | | | | | | | | |

| | | | | | | | |
|---|---|---|---|---|---|---|---|
| Screw Configuration B | 35 °C | | | | | | |
| | **C** | **K** | **C** | **K** | **C** | **K** | **C** |
| | 35 °C | | | | | | |

For each experiment, mean residence time, powder temperature, and powder bulk density were measured. The results are shown in Tables 2 and 3 below. "Physical Mix" refers to ibuprofen and poloxamer being combined but not subjected to heat and shear in the twin screw processor, and "Treated" refers to ibuprofen and poloxamer being subjected to the continuous melt-coating process disclosed herein.

**Table 2**

| | **Process** | **Mean Residence Time (seconds)** | **Temperature (°C)** |
|---|---|---|---|
| Ibuprofen 1 | 35°C, 150 rpm, 4 kneading zones | 44.35 | 25.2 |
| Ibuprofen 2 | 35°C, 150 rpm, 3 kneading zones | 31.23 | 24.0 |
| Ibuprofen 3 | 35°C, 150 rpm, 3 kneading zones | 41.34 | 23.9 |

**Table 3**

| | **Powder Bulk Density of 10% P407 Physical Mix (g/ml)** | **Powder Bulk Density of 10% P407 Treated (g/ml)** |
|---|---|---|
| Ibuprofen 1 | 0.320 | 0.365 |
| Ibuprofen 2 | 0.374 | 0.381 |
| Ibuprofen 3 | 0.382 | 0.368 |

### Example 2

Dissolution testing was performed on the following materials from Example 1 that incorporated each of Ibuprofen 1, Ibuprofen 2, or Ibuprofen 3 to determine the percentage of ibuprofen released over time:
- 10% Poloxamer 407 Treated
- 10% Poloxamer 407 Physical Mix
- 5% Poloxamer 407 Treated
- Ibuprofen, no surfactant
- 10% Poloxamer 188 Treated

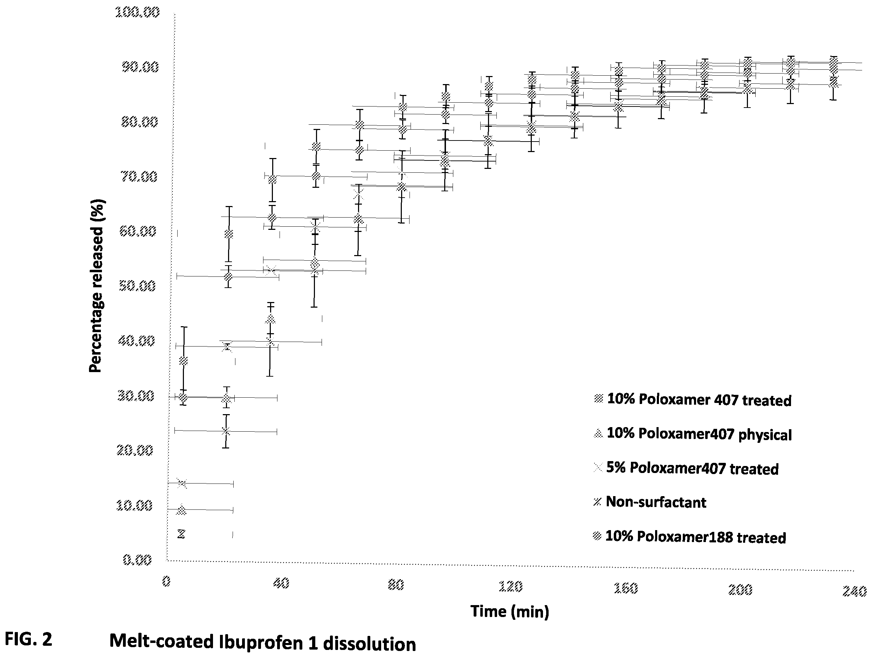

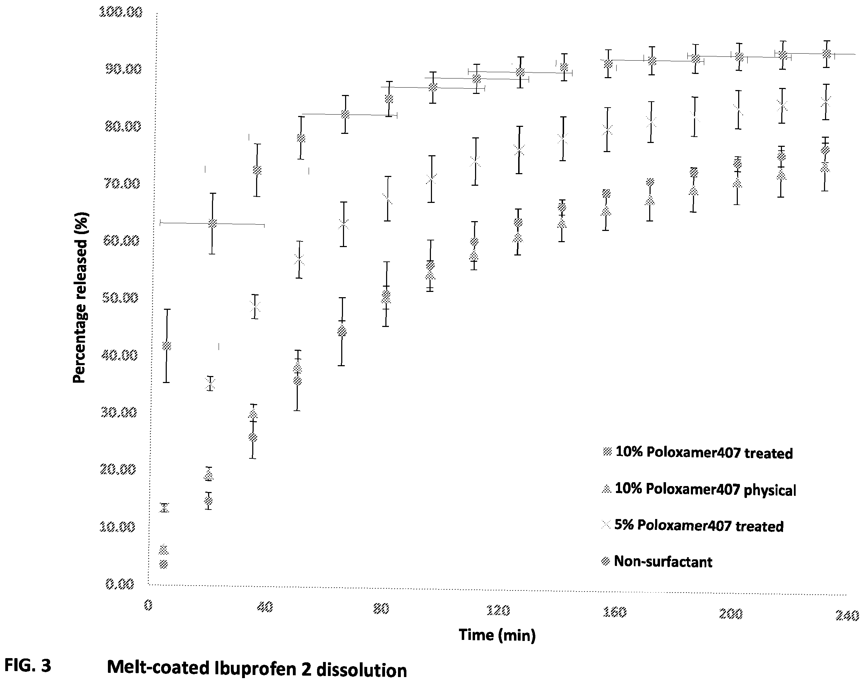

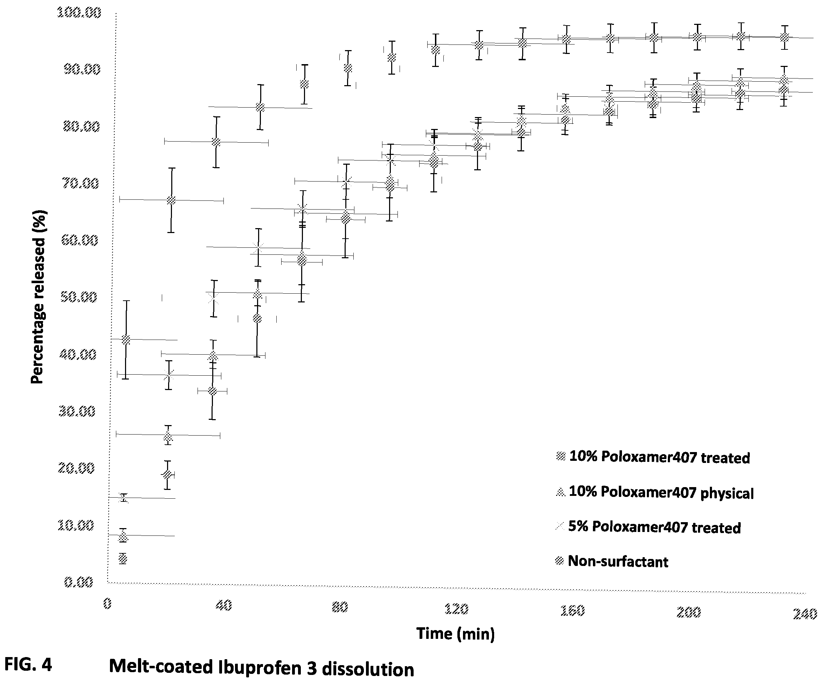

For each test, 12 mg of powder was added to a dissolution vessel, and dissolution was conducted using the USP paddle method at 75 rpm in 900 ml of a buffer medium comprising 0.02 M hydrochloric acid and having a pH of 1.8. The intrinsic solubility of the Ibuprofen powder was 0.053 mg/ml. The results of the dissolution testing for Ibuprofen 1 are shown in Figure 2. The results of the dissolution testing for Ibuprofen 2 are shown in Figure 3. The results of the dissolution testing for Ibuprofen 3 are shown in Figure 4.

Additionally, ibuprofen solid state characterization was conducted to assess the x-ray diffraction pattern of ibuprofen alone, ibuprofen melt-coated with poloxamer, and ibuprofen physically mixed with poloxamer. In each instance, all of the ibuprofen characteristic peaks were observed, which confirmed that the crystal form of ibuprofen was not changed by the melt-coating process.

Results: Uncoated ibuprofen consistently exhibited the slowest dissolution rate, and the physical mix of poloxamer and ibuprofen performed only slightly better. However, ibuprofen melt-coated with poloxamer exhibited a substantial and surprisingly dramatic improvement in dissolution rate, even when only a small amount of surfactant (e.g., 5 wt%) was melt-coated onto the ibuprofen particles. Hence, melt-coating provided significant advantages over both API alone and physical mixes of API and surfactant.

### Example 3

Dissolution testing was performed on three types of tablets prepared from the materials of Example 1, and having the formulations set forth in Table 4 below in order to determine the percentage of ibuprofen released over time:

**Table 4**

| **Ingredient** | **Treated** | **Physical Mix** | **No Surfactant** |
|---|---|---|---|
| Ibuprofen 2 | 15.9 mg (9.1 wt%) | 15.9 mg (9.1 wt%) | 15.9 mg (9.1 wt%) |
| Poloxamer 407 | 1.6 mg (0.9 wt%) | 1.6 mg (0.9 wt%) | -- |
| Avicel 102 | 49.0 mg (28 wt%) | 49.0 mg (28 wt%) | 49.0 mg (28 wt%) |
| Lactose 310 | 98.0 mg (56 wt%) | 98.0 mg (56 wt%) | 99.6 mg (56.9 wt%) |
| Crospovidone | 8.8 mg (5 wt%) | 8.8 mg (5 wt%) | 8.8 mg (5 wt%) |
| magnesium stearate (MgSt) | 1.7 mg (1 wt%) | 1.7 mg (1 wt%) | 1.7 mg (1 wt%) |
| **Total** | 175.0 mg | 175.0 mg | 175.0 mg |

The tablets were prepared by combining Ibuprofen 2, Poloxamer 407, Avicel 102, Lactose 310, and Crospovidone a V-blender (15 rpm for 15 min), and then adding MgSt (15 rpm for another 2 min). Tablets had a diameter of 8 mm and were prepared using a compaction force of 12 kN.

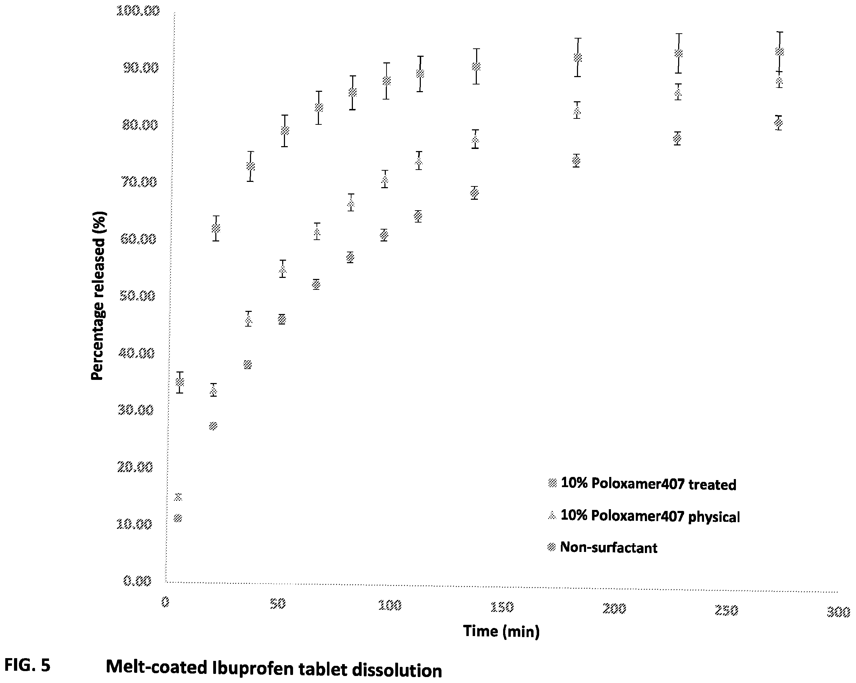

For each test, single tablets were added to a dissolution vessel, and dissolution was conducted using the USP paddle method at 75 rpm in 900 ml of a buffer medium comprising 0.02 M hydrochloric acid and having a pH of 1.8. The results of the dissolution testing are shown in Figure 5.

Results: Tablets prepared from uncoated ibuprofen exhibited the poorest dissolution rate, and tablets prepared from a physical mix of poloxamer and ibuprofen performed on only slightly better. However, tablets prepared from ibuprofen melt-coated with poloxamer exhibited a substantial and surprisingly dramatic improvement in dissolution rate. Hence, melt-coating provided significant advantages over both API alone and physical mixes of API and surfactant.

### Example 4

This example describes the preparation and analysis of ibuprofen alone and ibuprofen physically mixed with surfactant (polyoxyethylene stearate) as compared to ibuprofen melt-coated with polyoxyethylene stearate in accordance with the disclosed technology. In this example, Ibuprofen 2 (described above in Example 1)

Polyoxyethylene stearate generally functions as an emulsifying agent, solubilizing agent, and wetting agent. Polyoxyethylene stearate has a melting point of 53°C. The chemical structure of the polyoxyethylene stearate used in this example can be represented as:

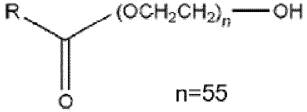

Pre-blends of polyoxyethylene stearate and ibuprofen were prepared using a 1 qt V-blender operated at 25 rpm for 15 minutes. Continuous melt-coating of the polyoxyethylene stearate onto the ibuprofen particles was carried out in an 11 mm Thermo Fisher twin screw extruder having 4 kneading zones, with 3 kneading elements in each zone, at a processing temperature of 50°C and an operational speed of 150 rpm, and having the screw configuration shown below:

| | | | | | | | | | |
|---|---|---|---|---|---|---|---|---|---|
| Screw Configuration | 50 °C | | | | | | | | |
| | **C** | **K** | **C** | **K** | **C** | **K** | **C** | **K** | **C** |
| | 50 °C | | | | | | | | |

Dissolution testing was performed on the following materials used or prepared herein in order to determine the percentage of ibuprofen released over time:
- 10% polyoxyethylene stearate Treated
- 10% polyoxyethylene stearate Physical Mix
- Ibuprofen, no surfactant

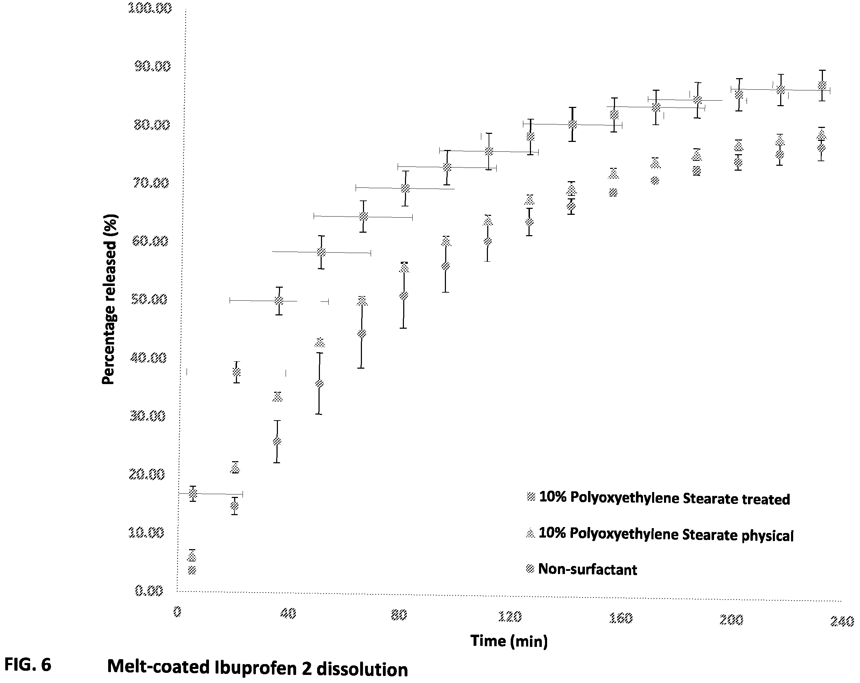

In each test, 12 mg of powder was added to a dissolution vessel, and dissolution was conducted using the USP paddle method at 75 rpm in 900 ml of a buffer medium comprising 0.02 M hydrochloric acid and having a pH of 1.8. The solubility of the powder was 0.053 mg/ml. The results of the dissolution testing are shown in Figure 6.

Results: Uncoated ibuprofen consistently exhibited the poorest dissolution rate, and the physical mix of polyoxyethylene stearate and ibuprofen performed only slightly better. However, ibuprofen melt-coated with polyoxyethylene stearate exhibited a substantial and surprisingly dramatic improvement in dissolution rate. Hence, melt-coating again was shown to provide significantly enhanced dissolution as compared to both API alone and physical mixes of API and surfactant.

### Example 5

This example describes the preparation and analysis of an API (carbamazepine) alone, and carbamazepine physically mixed with surfactant (cetylpyridinium chloride) as compared to carbamazepine melt-coated with cetylpyridinium chloride in accordance with the disclosed technology. Carbamazepine has low water solubility, a melting point of 189°C to 193°C, and a small particle size (D50 ≈ 13 micrometers). Cetylpyridinium chloride generally functions as a cationic surfactant, solubilizing agent, and wetting agent. Cetylpyridinium chloride has a melting point of 84°C to 86°C.

The chemical structures of carbamazepine and cetylpyridinium chloride are:

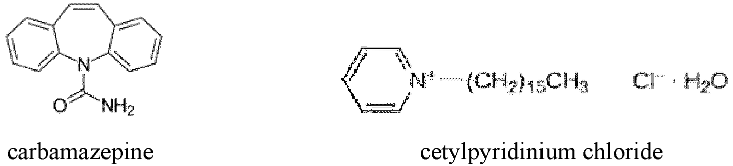

Pre-blends of cetylpyridinium chloride and carbamazepine were prepared using a V-blender operated at 25 rpm for 15 minutes. Continuous melt-coating of the cetylpyridinium chloride onto the carbamazepine particles was carried out in an 11 mm Thermo Fisher twin screw extruder having 3 kneading zones, with 3 kneading elements in each zone, at a processing temperature of 83°C and an operational speed of 150 rpm, and having the screw configuration shown below:

| | | | | | | | |
|---|---|---|---|---|---|---|---|
| Screw Configuration | 83 °C | | | | | | |
| | **C** | **K** | **C** | **K** | **C** | **K** | **C** |
| | 83 °C | | | | | | |

Dissolution testing was performed on the following materials used or prepared herein in order to determine the percentage of carbamazepine released over time:
- 10% cetylpyridinium chloride Treated
- 10% cetylpyridinium chloride Physical Mix
- Carbamazepine, no surfactant

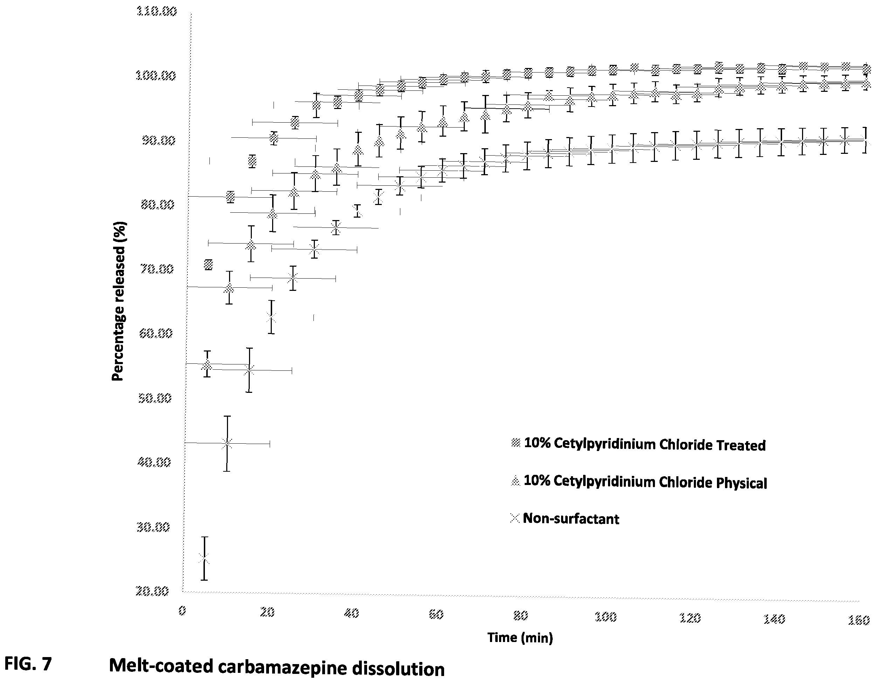

In each test, 50 mg of powder was added to a dissolution vessel, and dissolution was conducted using the USP paddle method at 100 rpm in 900 ml of a buffer medium of deionized water. For the "no surfactant" trials, a large volume of carbamazepine powder floated on top of the dissolution medium it took a considerable amount of time for the carbamazepine to become fully wetted by the medium. The results of the dissolution testing are shown in Figure 7.

Results: Uncoated carbamazepine exhibited the poorest dissolution rate, and the physical mix of cetylpyridinium chloride and carbamazepine performed somewhat better. However, carbamazepine melt-coated with cetylpyridinium chloride exhibited a substantial and surprisingly dramatic improvement in dissolution rate. Hence, yet again, even when a different API and a different surfactant were used, melt-coating was shown to provide significantly enhanced dissolution as compared to both API alone and physical mixes of API and surfactant.

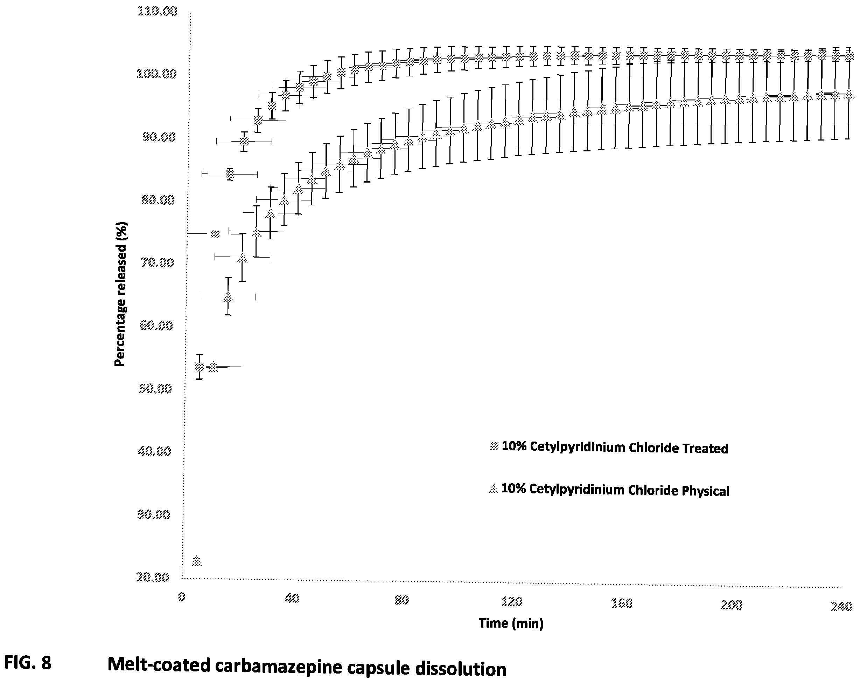

Further dissolution testing was performed on capsules prepared from 10% cetylpyridinium chloride Treated, and capsules prepared from 10% cetylpyridinium chloride Physical Mix in order to determine the percentage of carbamazepine released over time. For each test, capsules containing 50 mg of the corresponding powder were added to a dissolution vessel using a Japanese sinker to prevent capsules from sticking to the bottom of the vessel, and dissolution was conducted using the USP paddle method at 100 rpm in 900 ml of a buffer medium of deionized water. The results of the dissolution testing are shown in Figure 8.

Results: Capsules prepared from carbamazepine melt-coated with cetylpyridinium chloride exhibited a substantially faster dissolution rate as compared to capsules prepared from a physical mix of cetylpyridinium chloride and carbamazepine. Hence, this example shows that melt-coating surfactant onto API provides significant advantages over physical mixes of API and surfactant.

### Example 6

This example describes the preparation and analysis of tablets containing the API fenofibrate (particle size D50 = 353.7 µm; melting point = 81°C; manufacturer = LGM Pharma). The formulation is shown in Table 5, where surfactant was either excluded or the loading of surfactant was 10% w/w of API via melt-coating or physical mixing. Blends were produced using a LabRAM Acoustic Mixer (Resodyn Acoustic Mixers, Butte, MT) at 20% intensity for 40 s followed by additional 20 s after adding MgSt separately. Then, the blends were compressed into cylindrical tablets with flat surfaces and were 8mm in diameter at a compaction force of 7 kN. The mass of each tablet was about 150 mg.

**Table 5**

| | **Treated or Physical mix** | **Without Surfactant** |
|---|---|---|
| **Fenofibrate** | 60 mg (40%) | 60 mg (40%) |
| **Poloxamer407** | 6 mg (4%) | - |
| **Avicel102** | 37.5 mg (25%) | 37.5 mg (25%) |
| **Lactose310** | 37.5 mg (25%) | 43.5 mg (29%) |
| **Crospovidone** | 7.5 mg (5%) | 7.5 mg (5%) |
| **MgSt** | 1.5 mg (1%) | 1.5 mg (1%) |
| **Total** | 150.0 mg | 150 mg |

A dissolution test was performed for both unformulated powder (treated powder and physically mixed preblends) and finished products (tablets) by using a dissolution tester of apparatus II (Paddle type) (Agilent Technologies, Santa Clara, CA). 900 ml of the medium was added into each vessel and the temperature of the medium was maintained at 37.0 ± 0.5 °C throughout the dissolution test. The medium and the paddle speed conditions were as follows:
Medium: 0.7% (w/v) SLS solution
Paddle speed (rpm): 100
UV wavelength (nm): 287
m_{API} (mg): 50

At each pre-defined time point, 12 ml of solution was withdrawn by using a peristaltic pump and then pumped back to the vessel after measurement. The amount of released drug was analyzed using a Cary 60 UV-Vis spectrophotometer (Agilent Technologies, Santa Clara, CA) at the above-specified wavelength. For dissolution of unformulated powder, the mass of API-equivalent powder (m_{API}) was directly poured into the vessel at the starting point. Three replicates were implemented for dissolution of unformulated powder and six replicates were performed for finished products. Then, the dissolution profile was plotted as the percentage of drug release versus time. The cumulative % of drug release at the beginning of the dissolution (e.g., at 5 min (Q5min) or 20 min (Q20min)) and the time required to accomplish 80% drug release (T80%) were also documented. Moreover, a model independent approach was applied to compare dissolution profiles by calculating the mean dissolution time (MDT), which describes the mean time needed for the API molecule to be released from a solid dosage form.

The morphology of melt-coated API and physical mixture was visualized using a Zeiss Sigma field emission SEM (Carl Zeiss Microscopy, Jena, German). The powder samples were mounted on the aluminum pin stubs which were covered by carbon tape. Samples were pre-coated with conductive gold of 20 nm thickness by using an EMS150T ES turbo-pumped sputter coater (Electron Microscopy Sciences, PA, USA) to prevent the accumulation of static field. The secondary electrons (SE) were selected as signal detector. The beam energy was set to 5 kV and the sample was photographed at 500X magnification.

In addition, this system was equipped with an energy dispersive X-ray spectrometer wit h Oxford X-Max EDS detector (Oxford Instruments, MA, USA). The EDS imaging and spectra acquisition were implemented to obtain the chemical information of the sample by the Aztec software using in-built TruMap algorithm. In this study, the powders of interest were compressed into tablets measuring 8 mm in diameter at a low compression force (2 kN). The samples were also pre-coated with gold measuring 20 nm in thickness in the sputter coater. Then, the gold-coated tablets were mounted on the aluminum pin stubs covered by carbon tape so that the top surfaces were exposed for analysis. The bean energy was set to 10 kV. Then, the EDS images were obtained at the pixel-dwell time of 2000 us and the output count rate of 9500-12500 cps. Powder wettability was measured using drop penetration techniques, and the cosine of contact angle was determined as the index of wettability.

**Dissolution of treated powder:** First, the temperature of post-extruder powder was measured immediately at the exit of the extruder as 36°C for both screw configurations, which is far below the melting temperature of Fenofibrate (81°C), suggesting the low risk of API amorphization and degradation in coating process. It is noteworthy that the size of primary particles of Fenofibrate is significantly reduced when passing through the extruder, which is attributed to the milling of fragile particles at each kneading block. The mean particle size (D50) of post-extruder Fenofibrate, at both screw configurations was 94-96 µm in comparison to 353.7 µm for the as-received material. Due to the great change in particle size, the dissolution performance of post-extruder material would be different from pre-extruder material even if the API is not coated with surfactant. Therefore, in this study, we also implemented trials of specific conditions: the physical mixtures of API and surfactant were passed through the extruder at the same process parameters as melt-coating but using a cooled barrel (maintained at 25°C). The specific trials were expected to only have the particle size reduction without melt-coating occurring as there is no thermo-treatment being conducted in the process. In the discussion below, they are denoted as "untreated" trials to distinguish from the physical mixture with no extruder passage.

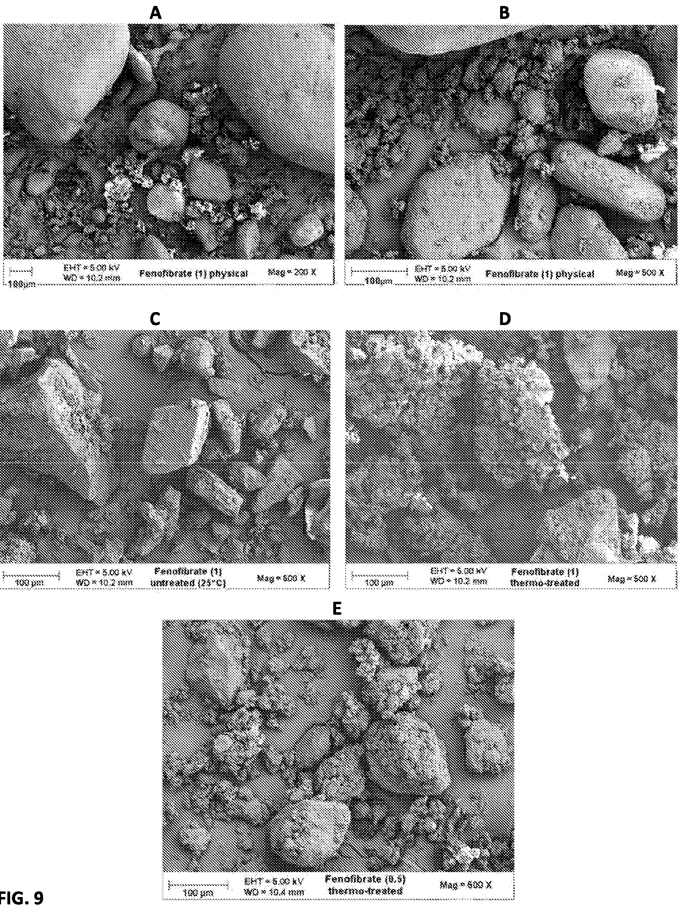

Figure 9 presents particle morphology by SEM for physical mixture, untreated mixture, and treated mixture at high (10% w/w) and low (5% w/w) surfactant loading. Apparently, in comparison to the physical mixture, the untreated mixture shows significant reduction in particle size, from spherical and rounded particles to angular and irregular particles. The smooth particle surface can be observed in both physical and untreated mixture. After the thermo-treatment, the mixture with high surfactant loading (10% w/w) exhibits the distinct surface, displaying new layers of coating on its originally smooth surface. The relatively less coating is observed when mixture contains lower amount of surfactant (5% w/w). Particles seem only partially coated or still remain uncoated. As a result, the SEM images evidence that the degree of coating greatly relates to the mass fraction of surfactant. In addition, it supports that the extruder passage leads to the breaking of particles in this specific case, but the coating does not occur when the mixture is only processed at a very low temperature. The localized melt of surfactant is the prerequisite for coating.

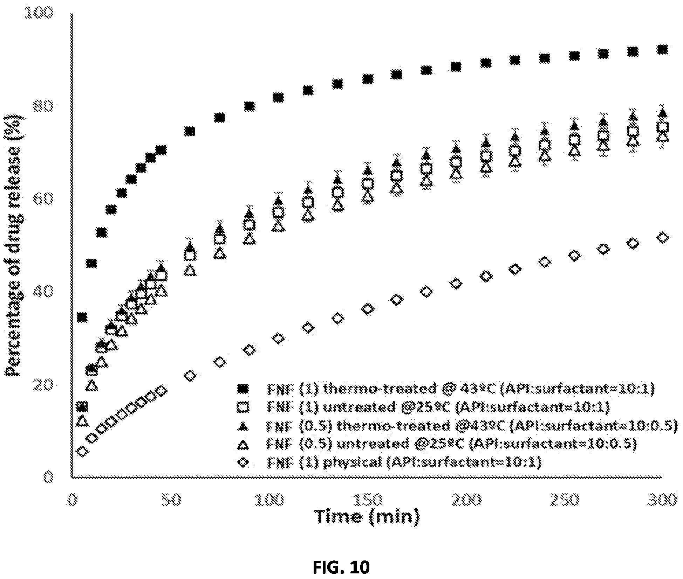

Next, the dissolution of treated powder, untreated powder and physical mixture were examined, as shown in Figure 10. Also, the extracted information from dissolution profiles is provided in Table 6, including the mean dissolution time, time needed for 80% drug release, and the percentage of drug release at 20min.

**Table 6**

| | **MDT (min)** | **T_{80%} (min)** | **Q₂₀ₘᵢₙ (%)** |
|---|---|---|---|
| **Fenofibrate (1) thermo-treated** | 36.6±0.5 | 95±8.6 | 56.6±2.3 |
| **Fenofibrate (1) untreated** | 66.2±0.6 | >300 | 31.7±0.7 |
| **Fenofibrate (1) physical** | 102.6±2.1 | >300 | 12.1±0.6 |

The most striking observation is that the treated powders (both high and low surfactant loading) displayed a substantially increased dissolution rate, as compared to the physical mixture. Note that, as mentioned, the primary crystal size of Fenofibrate is reduced as extruder passage. Both surface coating and reduction of API crystal size contribute to the enhancement. In contrast, for the untreated powders, the enhancement of dissolution is only attributed to crystal size reduction. Thus, it is more meaningful to compare the dissolution of treated powder with untreated powder to evaluate the effectivity of melt-coating. The results further indicate that the melt-coating of Fenofibrate with 10% w/w Poloxamer407 significantly enhances the dissolution rate in comparison to untreated powder, resulting in at least a three-fold faster release of more than 80% drug. It also nearly doubles the amount of drug release at the beginning of dissolution (at 20 min). It is also evident that the mean dissolution time of API-treated powder is greatly shorter than untreated one, which further shorter than physical mixture. On the other hand, when the Fenofibrate is coated with 5% w/w Poloxamer407, the enhancement is only marginal. Also, the variation of surfactant loading in untreated trials shows no effect on dissolution, which is attributed to that there is no interaction between surfactant and API, the untreated powder can be regarded as a physical mixture of milled-API with surfactant. In summary, the results confirm that the melt-coating of API with a certain amount of surfactant is able to considerably improve its dissolution.

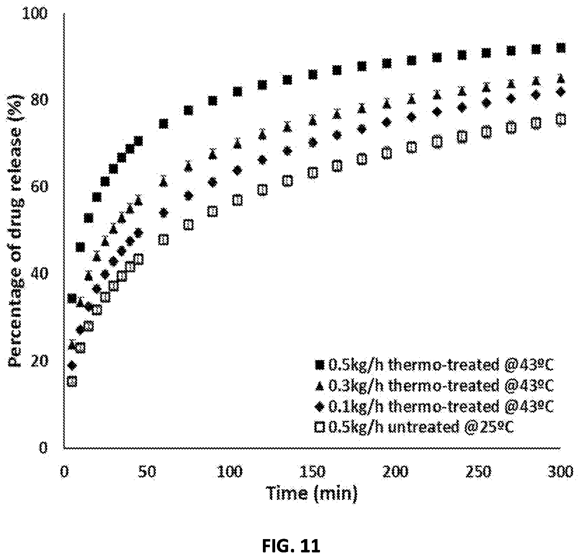

**Melt-coating at different screw-filling level:** This section describes a series of experiments implemented by varying the feed rate (0.1kg/h, 0.3kg/h and 0.5kg/h) while maintaining the constant screw speed (150rpm) and barrel temperature (43°C) for coating Fenofibrate with 10% w/w Poloxamer407. In this manner, the feed rate corresponds to the level of screw-filling, which further determines the degree of mechanical stress within the extruder. Figure 11 shows the dissolution of treated powder at different feed rates. For comparison, also plotted is the dissolution profile of the untreated powder. This graph indicates that all of the treated powders show improved dissolution. The coating process of higher screw-filling yields a greater dissolution improvement. A possible explanation of this phenomenon is that the larger mass of powder in each kneading block leads to a higher degree of localized compression and more intensive shearing being applied to particles. Additionally, it also causes the increased friction between powder and screw or barrel wall. These enhanced mechanical energies result in a high degree of surfactant melting and dispersive mixing, which further converts to a high degree of coating.

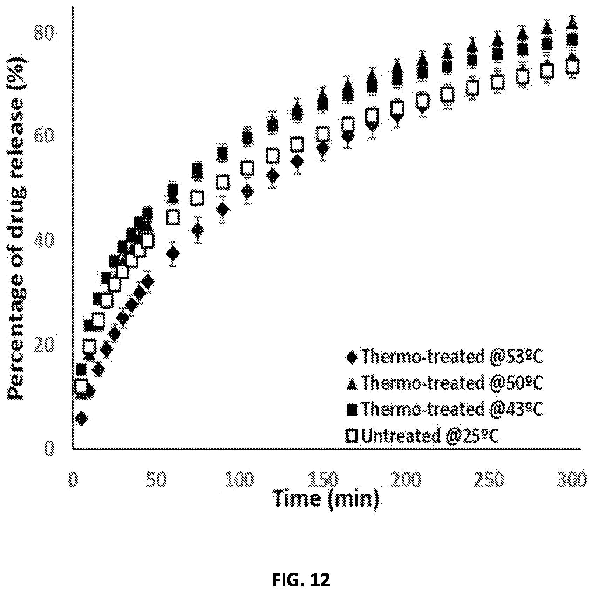

**Effect of temperature on melt-coating:** When the process is implemented in a cooled barrel (at 25°C), the dissolution of Fenofibrate can be much slower than the process in a relatively high barrel temperature. This section analyzes the effect of barrel temperature on melt-coating for trials containing 5% w/w Poloxamer407. Four temperature levels are discussed: 25°C, 43°C, 50°C, and 53°C, in which the highest temperature is at the melting point of Poloxamer407. As shown in Figure 12, processes with relatively high barrel temperature (43°C or 50°C) yield product with faster dissolution than the trial with low barrel temperature (25°C), although the enhancement of dissolution is likely modest due to an insufficient mass of surfactant for a higher level of coating. The result also indicates that increasing the barrel temperature to the melting point of surfactant does not yield the optimal dissolution, and instead causes a slightly delayed dissolution in comparison to the untreated powder. Especially at the beginning of the dissolution (<100min), a smaller amount of drug molecules is released at each sampling point. Such behavior can be interpreted as the formation of hard granules (or pellets) when the surfactant is completely melted. The formation of hard granules prevents dissolution medium from penetrating into the matrix of granules, which reduces the total area of surface that is fully exposed to the dissolution medium.

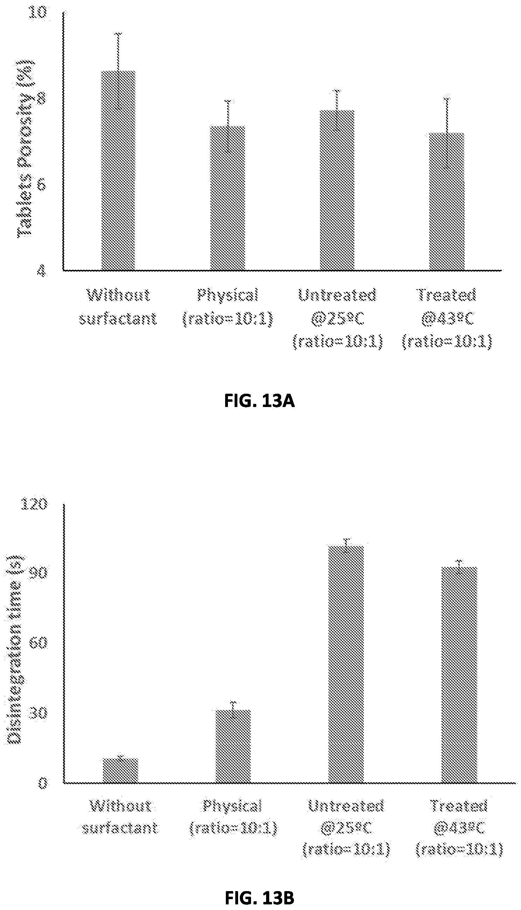

**Disintegration and dissolution of tablets (finished product):** This section describes a study of the performance of formulated tablets: 10% w/w Poloxamer407 treated, untreated, physically mixed Fenofibrate and the formulation without Poloxamer407. As each tablet contained a relatively large fraction of drug (40% w/w), tablet porosity and disintegration time may be significantly influenced by API-surfactant interaction (treated, physical or without surfactant). Therefore, the investigation of tablet performance includes the measurement of porosity and disintegration time. As shown in Figure 13A, no significant difference is observed in tablet porosity. Figure 13B presents the disintegration time, and shows that the tablets of both thermo-treated and untreated API yield a longer disintegration time than physical mixed ingredients, whereas the tablets without surfactant disintegrate immediately. This trend is at least partially due to the fact that the crystal size of treated and untreated API is much smaller than the API without extruder passage, which creates more available surface for bonding. In addition, the Poloxamer407 in the formulation has binding capacity when it is compressed into a tablet. All of the tablets completely disintegrate within 2 minutes, which indicates that the effect of disintegration on drug release throughout the dissolution is marginal.

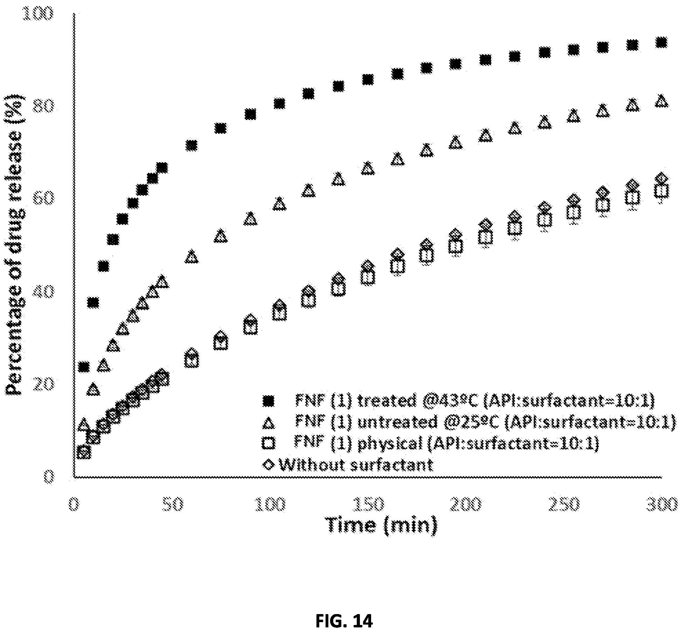

Dissolution profiles of the tablets are plotted in Figure 14. It is evident that the melt-coating of API with surfactant greatly improves the drug release of tablets in comparison to both untreated (extruder passage at low temperature) and physical mixed trials. It also shows that the physical addition of surfactant in formulation results in a comparable dissolution profile with no surfactant addition. As reported in Table 7, tablets present much shorter mean dissolution time when the API is thermo-treated with surfactant, which is able to release more than 80% drug within two hours. However, it requires five hours for untreated and much more than five hours for physically mixed tablets to approach 80% drug release. Moreover, within a short time range (20 min), the melt-coating allows the amount of drug release about two- and four-fold higher than untreated and physically mixed tablets, respectively.

**Table 7**

| **Summary of drug release from tablets (API : surfactant=10:1)** | | | |
|---|---|---|---|
| | **MDT (min)** | **T_{80%} (min)** | **Q₂₀ₘᵢₙ (%)** |
| **Fenofibrate (1) thermo-treated** | 44.4±0.8 | 105.0±0.0 | 51.2±0.2 |
| **Fenofibrate (1) untreated** | 73.8±1.1 | 282.5±11.3 | 28.5±0.7 |
| **Fenofibrate (1) physical** | 104.7±0.6 | >300 | 13.0±0.3 |

## Claims

1. A continuous process for melt-coating an active pharmaceutical ingredient, comprising:
(a) introducing an active pharmaceutical ingredient (API) and a surfactant into a twin-screw processor; and
(b) continuously heating and shearing the API and surfactant in the twin-screw processor at a processing temperature that is below the melting point of the surfactant and no lower than 10°C below the melting point of the surfactant so as to form melt-coated API particles comprising API particles with at least a partial coating of surfactant, wherein melt-coated API particles are formed, in which the surfactant coats 30% or more of the outer surface of the API particles, as determined by SEM images;
wherein the surfactant has a melting point of at least 10 degrees centigrade lower than the API melting point;
and wherein the melt-coated API particles comprise at least 90 wt% API based on the total weight of the melt-coated API particles.

2. The continuous process of claim 1, wherein the melt-coated API particles have faster dissolution as compared to a physical mix of the API and the surfactant, as determined by the time required to release 80% of the API in the melt-coated particles, when tested using any one of the following:
(1) a USP II apparatus, 900 ml vessel, 75 RPM, using deionized water;
(2) a USP II apparatus, 900 ml vessel, 75 RPM, using pH 2 simulated gastric fluid; or
(3) a USP II apparatus, 900 ml vessel, 75 RPM, using pH 6.8 buffer.

3. The continuous process of claim 1 or 2, wherein the process is operated under closed loop control using a combination of sensors, controllers, and actuators to maintain the process within a desired range of operating parameters.

4. The continuous process of any one of claims 1-3, wherein the twin-screw processor is an extruder, blender, mixer, or kneader.

5. The continuous process of any one of claims 1-4, wherein the API has an equilibrium solubility of less than 50 mg/ml in de-ionized water at 25 degrees centigrade.

6. The continuous process of any one of claims 1-5, wherein the API comprises one or more of ibuprofen, carbamazepine, fenofibrate, acetaminophen, indomethacin, flufenamic acid, imatinib, flufenamic acid, erlotinib hydrochloride, vitamin D, a steroid, estrodial, and a non-steroidal anti-inflammatory drug.

7. The continuous process of any one of claims 1-6, wherein the surfactant comprises one or more of poloxamer, polyoxyethylene stearate, cetylpyridinium chloride, polysorbate, and glyceryl monostearate.

8. The continuous process of any one of claims 1-7, wherein the process further includes: (c) formulating the melt-coated API particles into a finished pharmaceutical drug product, optionally wherein the finished pharmaceutical drug product is a solid oral dosage form, optionally wherein the solid oral dosage form is selected from a tablet, capsule, powder, and granulate.

9. The continuous process of claim 8, wherein step (c) further comprises combining the melt-coated API particles with one or more pharmaceutically acceptable excipients selected from carriers, fillers, extenders, binders, humectants, disintegrating agents, absorption accelerators, wetting agents, absorbents, lubricants, coloring agents, and diluents.

10. A pharmaceutical drug product comprising melt-coated API particles obtainable by the continuous process of any one of claims 1-9, and optionally at least one pharmaceutically acceptable excipient.

11. The pharmaceutical drug product of claim 10, wherein individual product units have faster dissolution than an individual product unit that differs only by having been made from a physical mix of the API and the surfactant, as determined by the time required to release 80% of the API in the product, when tested using any one of the following:
(1) a USP II apparatus, 900 ml vessel, 75 RPM, using deionized water;
(2) a USP II apparatus, 900 ml vessel, 75 RPM, using pH 2 simulated gastric fluid; or
(3) a USP II apparatus, 900 ml vessel, 75 RPM, using pH 6.8 buffer.

## Patentansprüche

1. Kontinuierlicher Prozess zum Schmelzbeschichten eines pharmazeutischen Wirkstoffs, umfassend:
(a) Einbringen eines pharmazeutischen Wirkstoffs (API) und eines Tensids in einen Doppelschneckenprozessor; und
(b) kontinuierliches Erhitzen und Scheren des API und des Tensids in dem Doppelschneckenprozessor bei einer Verarbeitungstemperatur, die unter dem Schmelzpunkt des Tensids und nicht niedriger als 10 °C unter dem Schmelzpunkt des Tensids ist, um schmelzbeschichtete API-Partikel zu bilden, die API-Partikel mit mindestens einer teilweisen Beschichtung aus Tensid umfassen, wobei schmelzbeschichtete API-Partikel gebildet werden, in denen das Tensid 30 % oder mehr der Außenoberfläche der API-Partikel beschichtet, wie durch REM-Bilder bestimmt;
wobei das Tensid einen Schmelzpunkt aufweist, der mindestens 10 Grad Celsius niedriger als der API-Schmelzpunkt ist;
und wobei die schmelzbeschichteten API-Partikel mindestens 90 Gew.-% API umfassen, bezogen auf das Gesamtgewicht der schmelzbeschichteten API-Partikel.

2. Kontinuierlicher Prozess nach Anspruch 1, wobei die schmelzbeschichteten API-Partikel eine schnellere Auflösung im Vergleich zu einer physikalischen Mischung des API und des Tensids aufweisen, wie bestimmt durch die Zeit, die erforderlich ist, um 80 % des API in den schmelzbeschichteten Partikeln freizusetzen, wenn unter Verwendung eines der Folgenden getestet wird:
(1) eine USP-II-Vorrichtung, 900-ml-Gefäß, 75 U/min, unter Verwendung von entionisiertem Wasser;
(2) eine USP-II-Vorrichtung, 900-ml-Gefäß, 75 U/min, unter Verwendung von simulierter Magenflüssigkeit mit pH 2; oder
(3) eine USP-II-Vorrichtung, 900-ml-Gefäß, 75 U/min, unter Verwendung eines Puffers mit pH 6,8.

3. Kontinuierlicher Prozess nach Anspruch 1 oder 2, wobei der Prozess unter Verwendung einer Kombination von Sensoren, Reglern und Aktuatoren in einem geschlossenen Regelkreis betrieben wird, um den Prozess innerhalb eines gewünschten Bereichs von Betriebsparametern zu halten.

4. Kontinuierlicher Prozess nach einem der Ansprüche 1 bis 3, wobei der Doppelschneckenprozessor ein Extruder, Mischer, Mixer oder Kneter ist.

5. Kontinuierlicher Prozess nach einem der Ansprüche 1 bis 4, wobei der API eine Gleichgewichtslöslichkeit von weniger als 50 mg/ml in entionisiertem Wasser bei 25 Grad Celsius aufweist.

6. Kontinuierlicher Prozess nach einem der Ansprüche 1 bis 5, wobei der API eines oder mehrere von Ibuprofen, Carbamazepin, Fenofibrat, Acetaminophen, Indomethacin, Flufenaminsäure, Imatinib, Flufenaminsäure, Erlotinib-Hydrochlorid, Vitamin D, einem Steroid, Estradiol und einem nichtsteroidalen entzündungshemmenden Arzneimittel umfasst.

7. Kontinuierlicher Prozess nach einem der Ansprüche 1 bis 6, wobei das Tensid eines oder mehrere von Poloxamer, Polyoxyethylenstearat, Cetylpyridiniumchlorid, Polysorbat und Glycerylmonostearat umfasst.

8. Kontinuierlicher Prozess nach einem der Ansprüche 1 bis 7, wobei der Prozess ferner einschließt: (c) Formulieren der schmelzbeschichteten API-Partikel zu einem fertigen pharmazeutischen Arzneimittelprodukt, optional wobei das fertige pharmazeutische Arzneimittelprodukt eine feste orale Darreichungsform ist, optional wobei die feste orale Darreichungsform ausgewählt ist aus einer Tablette, Kapsel, einem Pulver und Granulat.

9. Kontinuierlicher Prozess nach Anspruch 8, wobei Schritt (c) ferner das Kombinieren der schmelzbeschichteten API-Partikel mit einem oder mehreren pharmazeutisch verträglichen Hilfsstoffen umfasst, ausgewählt aus Trägern, Füllstoffen, Streckmitteln, Bindemitteln, Feuchthaltemitteln, Sprengmitteln, Absorptionsbeschleunigern, Netzmitteln, Absorptionsmitteln, Schmiermitteln, Farbstoffen und Verdünnungsmitteln.

10. Pharmazeutisches Arzneimittelprodukt, umfassend schmelzbeschichtete API-Partikel, erhältlich durch den kontinuierlichen Prozess nach einem der Ansprüche 1 bis 9, und optional mindestens einen pharmazeutisch verträglichen Hilfsstoff.

11. Pharmazeutisches Arzneimittelprodukt nach Anspruch 10, wobei einzelne Produkteinheiten eine schnellere Auflösung aufweisen als eine einzelne Produkteinheit, die sich nur dadurch unterscheidet, dass sie aus einer physikalischen Mischung des API und des Tensids hergestellt wurde, wie bestimmt durch die Zeit, die erforderlich ist, um 80 % des API in dem Produkt freizusetzen, wenn unter Verwendung eines der Folgenden getestet wird:
(1) eine USP-II-Vorrichtung, 900-ml-Gefäß, 75 U/min, unter Verwendung von entionisiertem Wasser;
(2) eine USP-II-Vorrichtung, 900-ml-Gefäß, 75 U/min, unter Verwendung von simulierter Magenflüssigkeit mit pH 2; oder
(3) eine USP-II-Vorrichtung, 900-ml-Gefäß, 75 U/min, unter Verwendung eines Puffers mit pH 6,8.

## Revendications

1. Procédé continu d'enrobage à l'état fondu d'un principe actif pharmaceutique, comprenant :
(a) l'introduction d'un principe actif pharmaceutique (API) et d'un agent tensioactif dans un processeur à vis jumelées ; et
(b) le chauffage et le cisaillement en continu de l'API et de l'agent tensioactif dans le processeur à vis jumelées à une température de traitement qui se trouve en dessous du point de fusion de l'agent tensioactif et est non inférieure à 10 °C en dessous du point de fusion de l'agent tensioactif de manière à former des particules d'API enrobées à l'état fondu comprenant des particules d'API avec au moins un enrobage partiel d'agent tensioactif, dans lequel des particules d'API enrobées à l'état fondu sont formées, dans lesquelles l'agent tensioactif enrobe 30 % ou plus de la surface externe des particules d'API, tel que déterminé par des images MEB ;
dans lequel l'agent tensioactif a un point de fusion inférieur d'au moins 10 degrés centigrades au point de fusion de l'API ;
et dans lequel les particules d'API enrobées à l'état fondu comprennent au moins 90 % en poids d'API par rapport au poids total des particules d'API enrobées à l'état fondu.

2. Procédé continu selon la revendication 1, dans lequel les particules d'API enrobées à l'état fondu ont une dissolution plus rapide par rapport à un mélange physique de l'API et de l'agent tensioactif, tel que déterminé par le temps nécessaire pour libérer 80 % de l'API dans les particules enrobées à l'état fondu, lors d'un test utilisant l'un quelconque de ce qui suit :
(1) un appareil USP II, cuve de 900 ml, 75 tr/min, utilisant de l'eau désionisée ;
(2) un appareil USP II, cuve de 900 ml, 75 tr/min, utilisant un fluide gastrique simulé de pH 2 ; ou
(3) un appareil USP II, cuve de 900 ml, 75 tr/min, utilisant un tampon pH 6,8.

3. Procédé continu selon la revendication 1 ou 2, dans lequel le procédé est mis en œuvre sous commande en boucle fermée, utilisant une combinaison de capteurs, dispositifs de commande, et actionneurs pour maintenir le procédé au sein d'une plage souhaitée de paramètres de fonctionnement.

4. Procédé continu selon l'une quelconque des revendications 1 à 3, dans lequel le processeur à vis jumelées est une extrudeuse, un mélangeur, un mixeur, ou un malaxeur.

5. Procédé continu selon l'une quelconque des revendications 1 à 4, dans lequel l'API a une solubilité à l'équilibre inférieure à 50 mg/ml dans de l'eau désionisée à 25 degrés centigrades.

6. Procédé continu selon l'une quelconque des revendications 1 à 5, dans lequel l'API comprend l'un ou plusieurs parmi ibuprofène, carbamazépine, fénofibrate, acétaminophène, indométhacine, acide flufénamique, imatinib, acide flufénamique, chlorhydrate d'erlotinib, vitamine D, un stéroïde, estrodial, et un médicament anti-inflammatoire non stéroïdien.

7. Procédé continu selon l'une quelconque des revendications 1 à 6, dans lequel l'agent tensioactif comprend l'un ou plusieurs parmi poloxamère, stéarate de polyoxyéthylène, chlorure de cétylpyridinium, polysorbate, et monostéarate de glycéryle.

8. Procédé continu selon l'une quelconque des revendications 1 à 7, dans lequel le procédé comporte en outre : (c) la formulation des particules d'API enrobées à l'état fondu en un produit médicamenteux pharmaceutique fini, facultativement dans lequel le produit médicamenteux pharmaceutique fini est une forme posologique orale solide, facultativement dans lequel la forme posologique orale solide est choisie parmi un comprimé, une capsule, une poudre, et un granulé.

9. Procédé continu selon la revendication 8, dans lequel l'étape (c) comprend en outre la combinaison des particules d'API enrobées à l'état fondu avec un ou plusieurs excipients pharmaceutiquement acceptables choisis parmi des supports, charges, agents diluants, liants, humectants, agents de désintégration, accélérateurs d'absorption, agents mouillants, absorbants, lubrifiants, agents colorants, et diluants.

10. Produit médicamenteux pharmaceutique comprenant des particules d'API enrobées à l'état fondu pouvant être obtenues par le procédé continu selon l'une quelconque des revendications 1 à 9, et facultativement au moins un excipient pharmaceutiquement acceptable.

11. Produit médicamenteux pharmaceutique selon la revendication 10, dans lequel des unités de produit individuelles ont une dissolution plus rapide qu'une unité de produit individuelle qui diffère uniquement en ce qu'elle a été préparée à partir d'un mélange physique de l'API et de l'agent tensioactif, tel que déterminé par le temps nécessaire pour libérer 80 % de l'API dans le produit, lors d'un test utilisant l'un quelconque de ce qui suit :
(1) un appareil USP II, cuve de 900 ml, 75 tr/min, utilisant de l'eau désionisée ;
(2) un appareil USP II, cuve de 900 ml, 75 tr/min, utilisant un fluide gastrique simulé de pH 2 ; ou
(3) un appareil USP II, cuve de 900 ml, 75 tr/min, utilisant un tampon pH 6,8.
